# EUROPEAN PATENT APPLICATION

(11) **EP 2 821 502 A1**
(43) Date of publication of application: **07.01.2015**
(21) Application number: 13305967.5
(22) Date of filing: 05.07.2013
(51) Int. Cl.: C12Q 1/68, G01N 33/68

(54) **Method for evaluating developmental competence of an oocyte**

(71) Applicant: Centre National de la Recherche Scientifique (CNRS), 75016 Paris (FR); INSTITUT NATIONAL DE LA RECHERCHE AGRONOMIQUE, 75007 Paris (FR); Universite Francois Rabelais De Tours, 37000 Tours (FR)
(72) Inventor: Puard, Vincent, 37000 Tours (FR); Royere, Dominique, 37300 Joue les Tous (FR); Reiter, Eric, 37380 Nouzilly (FR)
(74) Representative: Regimbeau

(57) **Abstract**

The present invention is relative to method for evaluating developmental competence of a mammalian oocyte comprising the step of assessing expression of at least one ovarian marker in a sample, preferably by RT-PCR or reverse phase protein array (RPPA) in samples of individual cumulus cells.

## Description

The present invention is relative to method for evaluating developmental competence of a mammalian oocyte comprising the step of assessing expression of at least one ovarian marker in a sample.

Defining the developmental ability of an embryo during in vitro fertilization remains a major challenge both in humans and domestic mammals. Morphological criteria are most frequently used to evaluate the developmental and implantation ability of the embryos in human assisted reproductive technology (ART). However such morphological criteria (zygote scoring, early cleavage and embryo morphology at day 2 or 3) remains poorly predictive of developmental or implantation ability (Guerif F. et al.: Does early morphology provide additional selection power 330 to blastocyst selection for transfer? Reproductive Biomedicine Online 2010, 21:510-519). Direct studies on embryos, such as genomic or proteomic analyses are difficult in human, since such approach remains invasive and might alter the embryo integrity (Jones GM et al., Novel strategy with potential to identify developmentally competent IVF, Hum Reprod 2008, 23:1748-1759.). Therefore ART laboratories need some indirect and non-invasive selection criteria, to easily and reproducibly select competent oocyte.

Various studies reported on molecules inside the follicle or the embryo microenvironment through proteomic and metabolomic analysis of oocytes or embryos (Royere D. et al., Non invasive assessment of embryo quality: proteomics, metabolomics and oocyte-cumulus dialogue, Gynecol Obstet Fertil 2009, 37:917-920) and have identify potential biomarkers of oocyte or embryo quality. Other studies focused on the somatic cells (cumulus [CCs] and/or granulosa cells [GCs]) surrounding the oocyte since their interactions are involved in the acquisition of oocyte meiotic and developmental competence (Gilchrist RB et al., Oocyte-secreted factors: regulators of cumulus cell 340 function and oocyte quality, Human Reproduction Update 2008, 14:159-177). Potential biomarkers of oocyte competence in humans and animals were proposed, based on specific genes expression in cumulus cells (Assou S. et al.; Human cumulus cells as biomarkers for embryo and pregnancy outcomes, Molecular Human Reproduction 2010, 16:531-538). Indeed, the fact that protein translation is a highly regulated process implies that mRNA abundance does not always correlate with the level of the corresponding proteins. Such discrepancy might be explained by differences in mRNA stability, degradation/synthesis rates or post-translational modification of the proteins. Therefore, the corresponding protein could lead to increased robustness and predictive value of biomarkers. Despite the fact that the ultimate effectors in cells remain the proteins, few studies addressed cumulus cells proteome analysis mainly because of a lack of sensitivity of the technics. Global proteins expression pattern of human individual cumulus cells was investigated by two-dimensional polyacrylamide gel electrophoresis according to ovarian stimulation protocol without identification of specific proteins linked to oocyte competence (Hamamah S., Comparative protein expression profiling in human cumulus cells in relation to oocyte fertilization and ovarian stimulation protocol, Reprod Biomed Online 2006, 13:807-814). Cumulus cells proteome was explored by mass spectrometry and Western Blot on pooled Cumulus cells and specific proteins implicated in fatty acid metabolism and pre-mRNA splicing according to maternal age were highlighted (McReynolds S. et al., Impact of maternal aging on the molecular signature of human cumulus cells, Fertility and Sterility 2012, 98:1574-1580.e1575). However, to date no study investigating individual cumulus cells proteome and specific proteins according to oocyte competence has been reported.

Surprisingly, the Inventors have found that the reverse-phase protein arrays (RPPA) method can be used to detect and analyse potential biomarkers related to oocyte developmental competence through a non-invasive procedure involving cumulus cells in human, and identified specific biomarkers. The Inventors have showed that an improved RPPA procedure, combining near infrared detection and signal amplification protocols lead to minimal background signal and exquisite signal/noise ratio to detect specific proteins in human cumulus cells. Even though antibody validation was a challenging problem, the Inventors managed to identify some antibodies useful for the reverse-phase protein arrays (RPPA) method.

Thus, the present invention provides a method for evaluating developmental competence of an oocyte or an embryo comprising the step of assessing the expression level of at least one ovarian marker in a sample, wherein said ovarian marker is selected from the group consisting of: *CUL4B, LGALS9, POLR3K, NFYβ, TRSPAP1, MERTK, POPDC2, PLCB1, SLC2A1 and FLJ11506* and combinations thereof, preferably in the group consisting of: *CUL4B, LGALS9, POLR3K, NFYβ, TRSPAP1 and MERTK,* and more preferably in the group consisting of: *CUL4B, POLR3K and MERTK.*

Preferably, the method of the invention is a method for evaluating developmental competence of an oocyte or an embryo comprising the step of assessing the expression level of at least one ovarian marker in a sample by:
a) assessing the expression level of at least one gene chosen in the group consisting of: *CUL4B, LGALS9, POLR3K, NFYβ, TRSPAP1, MERTK, POPDC2, PLCB1, SLC2A1 and FLJ11506,* preferably in the group consisting of: *CUL4B, LGALS9, POLR3K, NFYβ, TRSPAP1 and MERTK,* and more preferably in the group consisting of: *CUL4B and POLR3K*; or
b) assessing the expression level of at least one protein chosen in the group consisting of: RGS2, POLR3K and MERTK, preferably chosen in the group consisting of: POLR3K and MERTK.

By "ovarian marker" it should be understood particular genes and/or proteins expressed in ovarian follicles, in particular in cumulus cells.

Preferably, the ovarian marker is a cumulus cell marker. Said cumulus cell marker is expressed in cumulus cells originating from the ovarian follicle surrounding the oocyte to be tested.

As used herein, the term "sample" refers to any solid or liquid sample from a subject.

Preferably, the sample comprises follicular fluid, cumulus cells, polar bodies, oocytes, embryos or culture media in which the oocytes, cumulus cells, or embryos are cultured.

More preferably, the sample comprises cumulus cells, and even more preferably consists in cumulus cells.

Preferably, the oocytes, cumulus cells, and embryos are from mammal, preferably from human. Thus, preferably, the oocyte is mammalian oocyte and the embryo is a mammalian embryo, and more preferably the oocyte is a human oocyte and the embryo is a human embryo.

In a specific embodiment, the oocytes, cumulus cells, and embryos may be obtained from domesticated animals.

By "evaluating the developmental competence", it should be understood to assess the quality of an oocyte, i.e. to evaluate the chromosomal and genetic competence of the oocyte and its potential for fertilization, implantation and development.

The expression "cumulus cells" or "cumulus oophorus" refers to the cells surrounding the oocyte in an ovarian follicle and after ovulation.
Preferably, the method of the invention is for evaluating developmental competence of an oocyte.

By "oocyte", it should be understood a female gametocyte or germ cell involved in reproduction. In other words, "oocyte" refers to immature ovum, or egg cell.

By "embryo", it should be understood a multicellular diploid eukaryote in its earliest stage of development, from the time of first cell division. In humans, it should be understood a multicellular diploid eukaryote in its earliest stage of development, from the time of first cell division until about eight weeks after fertilization. Preferably, the embryo is selected from the time of first cell division to 1, 2, 3, 4, 5, 6, 7, 8, 9 or 10 days after fertilization.

In the meaning of the present invention, the term "subject" refers to an animal, preferably a mammal and more preferably a woman. More preferably, the subject is a woman in need of or receiving *in vitro* fertilization treatment.

Preferably, the method according to the present invention is an *in vitro* method.

Preferably, the genes used in the present invention are human genes and preferably their mRNA sequences are described in Table 1. One skilled in the art will of course select the appropriate sequences depending on the subject.

**Table 1**

| **Official symbol** | **Official Full Name** | **related ID for human mRNA** | **Forward sequence** | **Reverse sequence** |
|---|---|---|---|---|
| MERTK | c-mer proto-oncogene tyrosine kinase | NM_006343 (SEQ ID NO:1) | ATTCCCGATTGGAGACAGGAC | TCCATTCCCAGGGCAATATCC |
| | | | (SEQ ID NO: 16) | (SEQ ID NO: 17) |
| CUL4B | cullin 4B | NM_001079872 (SEQ ID NO:2) | GTCGGAAAGAGTGCATCTGTA | CAAGTTTGCTGGTGAAAGCA |
| | | | (SEQ ID NO: 18) | (SEQ ID NO: 19) |
| LGALS9 | Lectin, galactoside-binding, soluble, 9 | NM_002308 (SEQ ID NO:3) | CCAGGACGGACTTCAGATCA | CCACTGAAGCCAGTCTGAAA |
| | | | (SEQ ID NO: 20) | (SEQ ID NO: 21) |
| NFYB | nuclear transcription factor Y, beta | NM_006166 (SEQ ID NO:4) | GCTTTGACAGTTATGTGGAACC | TGACTGCTCCACCAATTCC |
| | | | (SEQ ID NO: 22) | (SEQ ID NO: 23) |
| PLCB1 | phospholipase C, beta 1 (phosphoinositide-specific) | NM_182734 (SEQ ID NO:5) | ACTTAAGCTGCAAGTCACTCCA | TCGCTTCCGATCTGCTGAAA |
| | | | (SEQ ID NO: 24) | (SEQ ID NO: 25) |
| SLC2A1 | solute carrier family 2 (facilitated glucose transporter), member 1 | NM_006516 (SEQ ID NO:6) | TCCCTGCAGTTTGGCTACAA | GGACCCATGTCTGGTTGTAGAA |
| | | | (SEQ ID NO: 26) | (SEQ ID NO: 27) |
| FLJ11506/ AAGAB | alpha- and gamma-adaptin binding protein | NM_001271886 (SEQ ID NO:7) | AGATGCCCAGGTTGATAGCA | CTCCAGTGGTAAGACTGGCTA |
| | | | (SEQ ID NO: 28) | (SEQ ID NO: 29) |
| POLR3K | polymerase (RNA) III (DNA directed) polypeptide K, 12.3 kDa | NM_016310 (SEQ ID NO:8) | CCCTACGTGCACAACATCAC | TCCACCAAGCACATCATCCA |
| | | | (SEQ ID NO: 30) | (SEQ ID NO: 31) |
| POPDC2 | popeye domain containing 2 | NM_022135 (SEQ ID NO:9) | GAGGGTGAGACACCCATCAAC | TAGTGCAGAAACTGCCCATCC |
| | | | (SEQ ID NO: 32) | (SEQ ID NO: 33) |
| TRNAU1AP | tRNA selenocysteine 1 associated protein 1 | NR_003109 (SEQ ID NO:10) | AAGGTGGTTTTGGACCAGAC | CTCGCTTCTGTTCCAGTTCA |
| | | | (SEQ ID NO: 34) | (SEQ ID NO: 35) |
| RGS2 | regulator of G-protein signaling 2, 24kDa | NM_002923 (SEQ ID NO: 11) | CAGAACGCAAGAAGGGAATAGGTG | TTTGGCACTCATAACGGACACTG |
| | | | ((SEQ ID NO: 36) | (SEQ ID NO: 37) |

Preferably, the protein sequencse are from human and corresponds to the following sequences: RGS2 (NP_002914.1, SEQ ID NO:12), POLR3K (NP_057394.2, SEQ ID NO: 13) and MERTK (AAI14918, SEQ ID NO: 14, or MERKT precursor NP_006334.2, SEQ ID NO: 15). One skilled in the art will of course select the appropriate sequences depending on the subject.

The method of the present invention permits to differentiate oocytes or embryo competent for implantation from oocytes or embryo not competent for implantation.

In a preferred embodiment, the method of the invention comprises a step of comparing the expression level of at least one ovarian marker with a control expression level (control/reference value).

Thus, according to a particular aspect of the invention, the method comprises a further step of concluding that the oocyte or the embryo is competent if there is a differential level of expression of ovarian marker between the test sample and the control value.

Preferably, the "control value" or the "control expression level" is the level of expression of the ovarian marker in cumulus cells from an oocyte yielding to a blastocyst after 5 or 6 days of *in vitro* culture (positive control) or the level of expression of the ovarian marker in cumulus cells from an oocyte arresting development at the embyo stage after 5 or 6 days of *in vitro* culture (negative control).

Preferably, the method of the invention comprises a further step of selecting the tested oocyte if there is a differential level of expression of ovarian marker between the test sample and the control value.

"Differential level of expression" means a significantly higher or lower expression level of said gene/protein compared to a control expression level.

"Increased expression" means a significantly higher expression level of said gene/protein compared to a control expression level. Preferably, "increased expression" means an expression level in a biological sample that is greater by at least 5% than the control expression level of said gene/protein, preferably greater by at least 10%, 15%, 20%, 30%, 40, 50% than the control expression level of said gene/protein, and even more preferably greater by at least 90% than the control expression level of said gene/protein.

"Decreased expression" means a significantly lower expression level of said gene/protein compared to a control expression level. Preferably, "decreased expression" means an expression level in a biological sample that is lower by at least 5% than the control expression level of said gene/protein, preferably lower by at least 10%, 15%, 20%, 30%, 40, 50% than the control expression level of said gene/protein, and even more preferably greater by at least 90% than the control expression level of said gene/protein.

According to a specific aspect, the method includes a further step of obtaining the sample from the subject, in particular obtaining the cumulus cells, before the step of assessing the expression level of at least on ovarian marker.

In one embodiment, the method of the present invention is completed by a further step of detecting and/or assessing the expression level of other biomarkers in follicular fluid, cumulus cells, polar bodies or culture media in which the oocytes, cumulus cells, or embryos are cultured.

Preferably, the expression level of at least said one ovarian marker is assessed by determining the expression level of mRNA transcripts or mRNA precursors of at least one gene chosen in the group consisting of: *CUL4B, LGALS9, POLR3K, NFYβ, TRSPAP1, MERTK, POPDC2, PLCB1, SLC2A1 and FLJ11506* and combination thereof. More preferably, said gene is chosen in the group consisting of: *CUL4B, LGALS9, POLR3K, NFYβ, TRSPAP1 and MERTK,* and even more preferably in the group consisting of: *CUL4B* and *POLR3K.*

In a particular embodiment, the differentially expressed gene is *CUL4B* and the increased expression level of said gene compared to the control value is associated with good developmental competence, whereas a decreased expression level of said gene compared to the control value is associated with bad developmental competence.

In a particular embodiment, the differentially expressed gene is chosen in the group consisting of: *LGALS9, POLR3K, NFYβ, TRSPAP1, MERTK, POPDC2, PLCB1, SLC2A1 and FLJ11506,* preferably in the group consisting of: *LGALS9, POLR3K, NFYβ, TRSPAP1 and MERTK,* and even more preferably is *POLR3K* and the increased expression level of said gene compared to a control value is associated with bad developmental competence, whereas a decreased expression level of said gene compared to a control value is associated with good developmental competence.

Furthermore, the Inventors have shown that the level of expression of the proteins RGS2, POLR3K and MERTK was significantly increased in cumulus cells from an oocyte yielding to a blastocyst after 5 or 6 days of *in vitro* culture (CC_{B+}) compared to cumulus cells from an oocyte arresting development at the embyo stage after 5 or 6 days of *in vitro* culture (CC_{B-}).

Thus, in a particular embodiment, the expression level of at least said one ovarian marker is assessed by determining expression level of at least a protein chosen in the group consisting of: RGS2, POLR3K and MERTK, preferably in the group consisting of: POLR3K and MERTK.

Preferably, an increased level of expression of said at least one protein chosen in the group consisting of: RGS2, POLR3K and MERTK, compared to a control value is associated with good developmental competence, whereas a decreased level expression compared to a control value is associated with bad developmental competence.
In one embodiment, the method according to the invention comprises a step of assessing the level expression of at least 2, 3, 4, 5, 6, 7, 8, 9, 10 or 11 ovarian markers chosen in the group consisting of: *CUL4B, LGALS9, POLR3K, NFYβ, TRSPAP1, MERTK, RGS2, POPDC2, PLCB1, SLC2A1 and FLJ11506,* preferably at least 2, 3, 4, 5, 6 or 7 ovarian markers chosen in the group consisting of: *CUL4B, LGALS9, POLR3K, NFYβ, TRSPAP1, RGS2 and MERTK.*

Preferably, the method according to the invention comprises a step of assessing the expression level of mRNA transcripts or mRNA precursors of at least 2, 3, 4, 5, 6, 7, 8, 9, 10 or 11 genes chosen in the group consisting of: *CUL4B, LGALS9, POLR3K, NFYβ, TRSPAP1, MERTK, RGS2, POPDC2, PLCB1, SLC2A1 and FLJ11506.*

Preferably, the method according to the invention comprises a step of assessing the expression level of at least 2 or 3 proteins chosen in the group consisting of: RGS2, POLR3K and MERTK.

In a particular embodiment, the method according to the invention comprises the steps of:
a) assessing the expression level of at least 1, 2, 3, 4, 5, 6, 7, 8, 9, 10 or 11 genes chosen in the group consisting of: *CUL4B, LGALS9, POLR3K, NFYβ, TRSPAP1, MERTK, RGS2, POPDC2, PLCB1, SLC2A1 and FLJ11506;* and
b) assessing the expression level of at least 1, 2 or 3 proteins chosen in the group consisting of: RGS2, POLR3K and MERTK.

Preferably, in the method according to the present invention, the expression level of the ovarian marker is determined by measuring the expression level of the polypeptides encoded by said gene or a fragment thereof, or by determining the expression level of the mRNA from said gene or a fragment thereof.

In one particularly preferred embodiment, the expression level of each of the genes is determined by analysis of the expression of mRNA transcripts or mRNA precursors, such as a native RNA, of said gene. Said analysis can be done by preparing mRNA/cDNA from cells of a patient's biological sample, and hybridizing the mRNA/cDNA with a reference polynucleotide. The prepared mRNA/cDNA may be used in analysis by hybridization or amplification that includes, without being limiting, Southern and Northern analysis, PCR ("polymerase chain reaction"), such as quantitative PCR (Taqman) and the use of probes ("probe arrays") such as GeneChip® DNA matrices ®(AFFYMETRIX). Preferably, the expression level of each of the genes is determined by quantitative polymerase chain reaction (qPCR, also known as real-time PCR).

Advantageously, the analysis of the mRNA expression transcript of the genes involves a nucleic acid amplification process, such as, for example, RT-PCR (experimental method described in US patent 4,683,202), ligase chain reaction (BARANY, Proc. Natl. Acad. Sci. USA, vol. 88, p: 189-193, 1991), self sustained sequence replication (GUATELLI et al., Proc. Natl. Acad. Sci. USA, vol. 87, p: 1874-1878, 1990) and transcriptional amplification system. (KWOH et al., Proc. Natl. Acad. Sci. USA, vol. 86, p: 1173-1177, 1989), "Q-Beta Replicase" (LIZARDI et al., Biol. Technology, vol. 6, p: 1197, 1988), "rolling circle replication" (U. S. Patent No. 5,854,033) or any other nucleic acid amplification method, followed by a step of detecting the amplified molecules by techniques well known to the skilled person. These detection modes are particularly useful for detecting nucleic acid molecules in very small quantities. Thus, according to a preferred embodiment, the method according to the present invention comprises an additional step of amplifying the mRNA or cDNA of the genes, the complementary sequence thereof or a fragment thereof.

Such as used here, amplification primers are defined as being a pair of nucleic acid molecules that can respectively pair with the 3' and 5' regions of a gene in a specific manner (positive and negative strands or vice versa) and encompassing a short region of said gene. Generally, amplification primers have a length of 10 to 30 nucleotides and allow amplifying a region of a length comprised between 50 and 200 nucleotides. Advantageously the primers used in the present invention are those listed in Table 1.

In another particularly preferred embodiment, the expression level of the genes is assessed by determining the expression level of the polypeptide/protein encoded by said gene or a fragment thereof. Said analysis can be done by using an antibody (for example, a radiolabeled antibody, an antibody labeled with a chromophore, a fluorophore or an enzyme) an antibody derivative (for example, an antibody conjugated to a substrate or to a protein, or a ligand of a protein of a ligand/protein pair (for example biotin-streptavidin) or an antibody fragment (for example, a single chain antibody, a hypervariable domain of an isolated antibody, etc.) which specifically binds the polypeptide encoded by said gene. In a particular embodiment, the antibody can one of those disclosed in table 4.

Said analyses can be done by many techniques known to the skilled person including, without being limiting, immunological tests based on the use of enzymatic activity ("enzyme immunoassay" EIA), immunological tests based on the use of radioactive isotopes (RIA), western blot analysis and ELISA ("enzyme linked immunosorbent assay") tests and Reverse Phase Protein Array (RPPA). Preferably, the expression level of the protein is determined by Reverse Phase Protein Array (RPPA). More preferably, the expression level of the protein is determined by Reverse Phase Protein Array (RPPA) combined with near-infrared (NIR) dyes.

Reverse Phase Protein Array is a sensitive and quantitative technique allowing the detection of specific proteins from very small quantities of biological samples. This technique leads to minimal background signal and exquisite signal/noise ratio.
This technique also presents the advantage to be cost and material effective. Nevertheless, this technique necessitates some specific antibodies to specifically detect the protein/polypeptides of interest.

Interestingly, the Inventors of the present invention identified specific antibodies to detect the proteic ovarian markers of the invention (see table 4 of example 4).

Consequently, according to a preferred embodiment, the expression level of the protein is determined by Reverse Phase Protein Array (RPPA) using the appropriate antibodies disclosed in table 4.

In the meaning of the present invention, "polypeptide" means a sequence comprising at least two amino acids, and the terms "polypeptide", "peptide" and "protein" may be used interchangeably.

In the meaning of the present invention, "mRNA or cDNA fragment" means a sequence of at least 50 nucleic acids, for example at least 100 or 150 nucleic acids, preferably at least 200 nucleic acids, for example at least 250 or 350 nucleic acids, and in a particularly preferred manner, of at least 400 nucleic acids.

In the meaning of the present invention, "polypeptide fragment" means a sequence of at least 50 amino acids, for example at least 100 or 150 amino acids, preferably at least 200 amino acids, for example at least 250 or 350 amino acids, and in a particularly preferred manner, a polypeptide of at least 400 amino acids.

Preferably, the method according to the present invention may be useful in assisted reproduction techniques, i.e. wherein oocytes and/or embryos are manipulated. For example, said assisted reproduction techniques comprises *in vitro* fertilization, intracytoplasmic sperm injection, embryo transfer and long-term storage.

The method according to the present invention may also be useful oocyte long-term storage for later use. For example, long-term storage can be done by freezing.
Preferably, said method is being performed before implantation, before fertilization, to assist in maximizing the generation of chromosomally normal embryos or to assist in minimizing the generation of chromosomally abnormal embryos.

According to another aspect, the present invention is relative to a method of assisted reproduction, comprising a step of evaluating developmental competence of the oocyte as described above.

Said method of assisted reproduction can further include at least one of the steps comprised in the group consisting of: *in vitro* fertilization, intracytoplasmic sperm injection, embryo transfer and long-term storage.

According to another aspect, the present invention is relative to a method of oocyte or embryo conservation, comprising a step of evaluating developmental competence of said oocyte or embryo as described above. Said method of conservation can include a subsequent step of freezing the selected oocyte or embryo.

According to another aspect, the invention is also relative to method for selecting embryo to be transferred to a subject, comprising the comprising a step of evaluating developmental competence of the oocyte giving rise to said embryo or of said embryo as defined above.

By the "oocyte giving rise to said embryo", one should understand an oocyte which by fertilization becomes said embryo to be selected.

Preferably, said method comprises a step of assessing the expression level of at least one ovarian marker as defined above.

Said non-invasive and non-damaging method permits to reduce the need of multiple embryo transfer while maximizing pregnancy rates.

According to another aspect, the present invention is relative to a method to evaluate the efficiency of a treatment of a patient comprising the step of assessing the expression level of at least one ovarian marker as defined above.

Preferably, said treatment is a hormonal treatment

For instance, said treatment may be chosen in the group consisting of: a follicle-stimulating hormone (FSH) treatment, luteinizing hormone (LH) treatment, Gonadotropin-releasing hormone (GnRH) treatment, Gonadotropin-releasing hormone analogs treatment or combination thereof.

Alternatively, said treatment may be a contraceptive treatment.

According to another aspect, the present invention relates to an *in vitro* method for screening compounds capable to stimulate or to inhibit the oocyte competence, comprising the steps of:
a) contacting at least one cumulus cell with a compound to be screened for activity to stimulate or inhibit the competence of an oocyte; and
b) determining an expression level of at least one ovarian marker in said at least one cumulus cell contacted with said compound, wherein said at least one ovarian marker is selected from the group consisting of: CUL4B, LGALS9, POLR3K, NFYβ, TRSPAP1, MERTK, RGS2, POPDC2, PLCB1, SLC2A1 and FLJ11506 and combinations thereof, preferably in the group consisting in CUL4B, LGALS9, POLR3K, NFYβ, TRSPAP1 and MERTK and combinations thereof.

In a particular embodiment, said method can include a further step c) of comparing the expression level of said at least one ovarian marker measured in step b) with the expression level of said at least cumulus cells non-contacted with the compound to be screened.

In a particular embodiment, said method can include a further step d) of concluding that, if a difference in the expression level is observed, it is indicative of the compound stimulatory or inhibitory effect.

Alternatively, the method for screening compounds capable to stimulate or inhibit the oocyte competence can be an *in vivo* method, and can for example comprise the steps of:
a) administrating the compound to be screened to a subject;
b) determining an expression level of at least one ovarian marker in at least one cumulus cell, wherein said at least one ovarian marker is selected from the group consisting of: CUL4B, LGALS9, POLR3K, NFYβ, *TRSPAP1,* MERTK, RGS2, POPDC2, PLCB1, SLC2A1 and FLJ11506 and combinations thereof, preferably in the group consisting in CUL4B, LGALS9, POLR3K, NFYβ, TRSPAP1 and MERTK and combinations thereof.

In a particular embodiment, said method can include a further step c) of comparing the expression level of said at least one ovarian marker measured in step b) with a control expression level.

According to another aspect, the present invention relates to the use of the expression level of at least one ovarian marker chosen in the group consisting of: : *CUL4B, LGALS9, POLR3K, NFYβ, TRSPAP1, MERTKPOPDC2, PLCB1, SLC2A1 and FLJ11506* and combinations thereof, preferably at least one, preferably at least 2, at least 3, at least 4, at least 5 or 6 genes chosen in the group consisting of: : *CUL4B, LGALS9, POLR3K, NFYβ, TRSPAP1 and MERTK* and combinations thereof, for evaluating developmental competence of a oocyte.

Preferably, the invention relates to the use of the expression level of at least one gene chosen in the group consisting of: *CUL4B, LGALS9, POLR3K, NFYβ, TRSPAP1, MERTK, RGS2, POPDC2, PLCB1, SLC2A1 and FLJ11506,* preferably in the group consisting of: *CUL4B, LGALS9, POLR3K, NFYβ, TRSPAP1 and MERTK,* and more preferably in the group consisting of: *CUL4B and POLR3K* or of the expression level of at least one protein chosen in the group consisting of: RGS2, POLR3K and MERTK, preferably chosen in the group consisting of: POLR3K and MERTK for evaluating developmental competence of a oocyte.

The present invention also relates to a kit for evaluating developmental competence of a oocyte comprising means for determining the expression level of at least one, preferably at least 2, at least 3, at least 4, at least 5, at least 6, at least 7, at least 8, at least 9 or 10 ovarian markers chosen in the group consisting of: *CUL4B, LGALS9, POLR3K, NFYβ, TRSPAP1, MERTK, POPDC2, PLCB1, SLC2A1 and FLJ11506* and combinations thereof, preferably at least one, preferably at least 2, at least 3, at least 4, at least 5 or 6 genes chosen in the group consisting of: : *CUL4B, LGALS9, POLR3K, NFYβ, TRSPAP1 and MERTK* and combinations thereof.
Preferably, said kit can comprise a pair of primers for amplifying at least one gene chosen from the group consisting of: *CUL4B, LGALS9, POLR3K, NFYβ, TRSPAP1, MERTK, POPDC2, PLCB1, SLC2A1 and FLJ11506* and combinations thereof, preferably chosen in the group consisting of: *CUL4B, LGALS9, POLR3K, NFYβ, TRSPAP1 and MERTK.*

In a particularly preferred manner, said at least one primer pair is chosen in Table 1.

Alternatively, said kit can comprise means for determining the expression level of at least one, preferably at least 2, and more preferably at least 3 proteins chosen in the group consisting of: RGS2, POLR3K and MERTK, preferably chosen in the group consisting of: POLR3K and MERTK.

Preferably, said kit can comprise at least one antibody which specifically binds to a protein chosen in the group consisting of: RGS2, POLR3K and MERTK, preferably chosen in the group consisting of: POLR3K and MERTK.

Preferably, said antibody is chosen in Table 4 below.

### BRIEF DESCRIPTION OF THE FIGURES

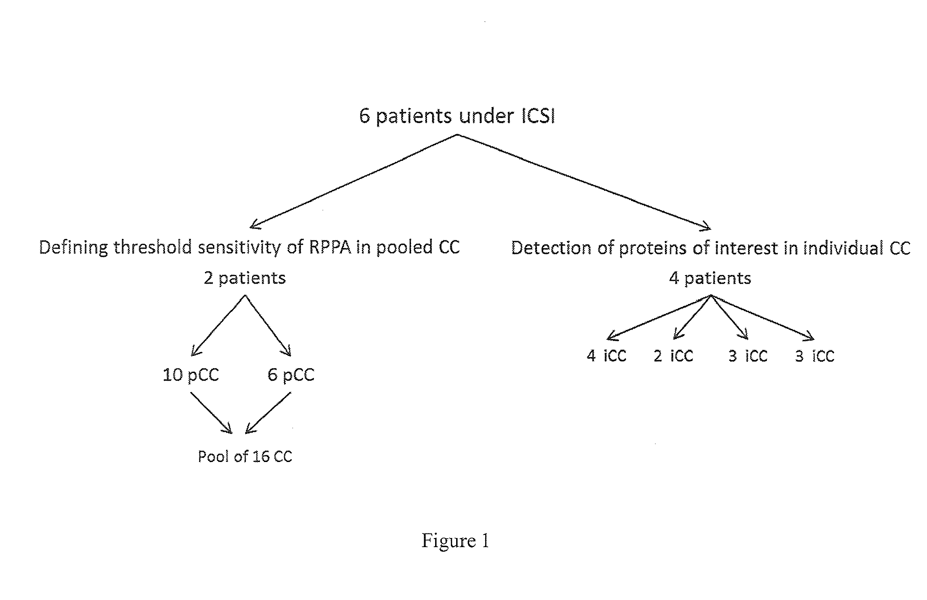

Figure 1 illustrates distribution of patients included in study. Cumulus cells (CC) of 2 patients were included for defining the threshold of sensitivity of RPPA. After individual recovery, the CC of a same patient were pooled (pCC) before be pooled together. Thirteen individual CC (iCC) of 4 patients were used to test the ability of RPPA to detect specific proteins of interest.

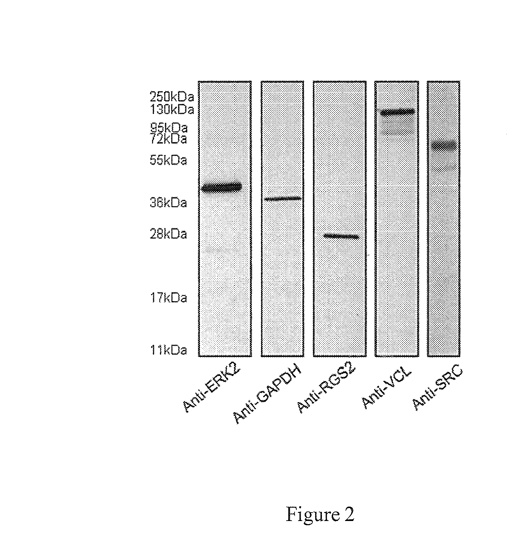

Figure 2 shows specificity of antibodies. HEK293 cells lysates were analysed by Western blot to test the specificity of antibodies.

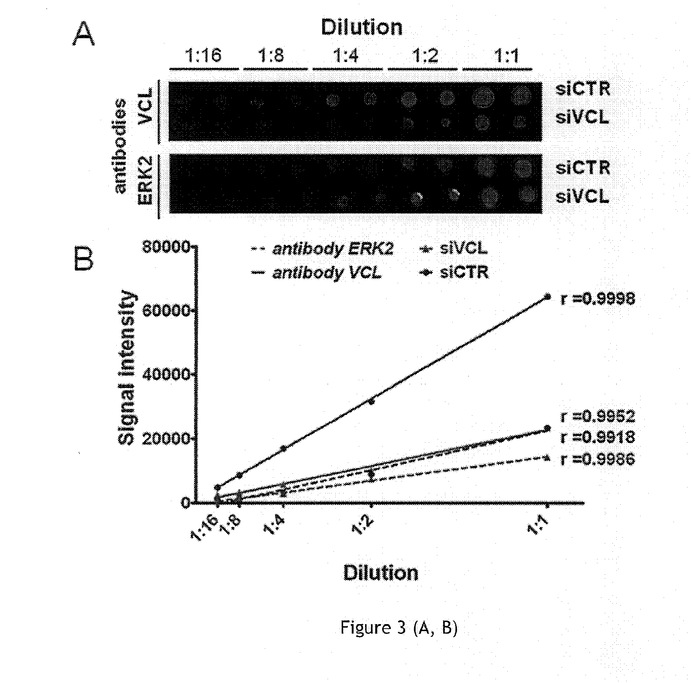

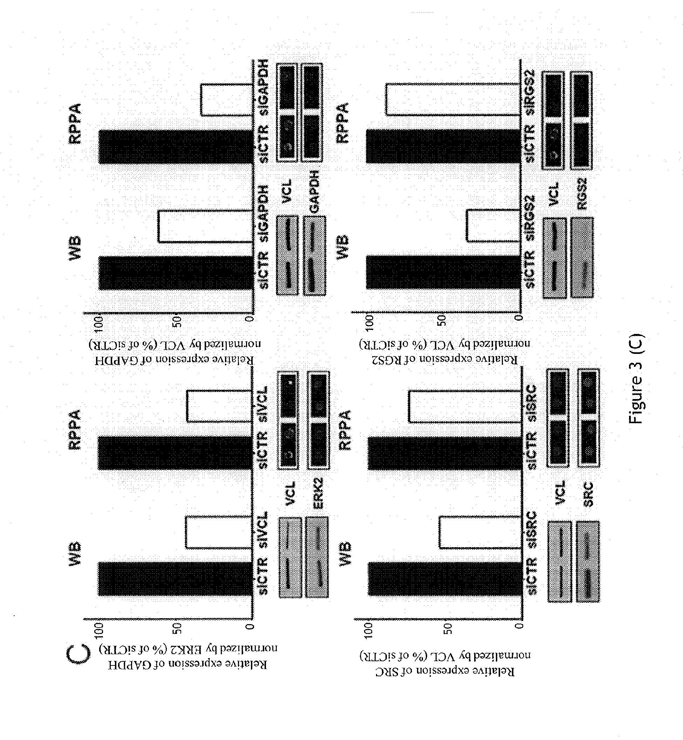

Figure 3 shows knockdown efficiencies of target proteins in HEK293 cells measured by Reverse Phase Protein Array (RPPA) and Western blotting (WB). Figure 3 A shows detection by RPPA of VCL and ERK2 in serial dilution of HEK293 cells transfected by siRNA targeting VCL (siVCL) or non-silencing siRNA control (siCTR). Figure 3B shows linear regression was performed for ERK2 and VCL protein and Pearson correlation between dilution and the signal intensity was calculated for each conditions of figure 2A. Signal intensities were expressed as mean of the replicates C. Level of expression by WB and RPPA of targeted proteins in HEK293 cells transfected by siRNA targeting proteins of interest or non-silencing siRNA control (siCTR). Specific bands for WB and spots corresponding to the dilution of the dynamic range in RPPA were presented.

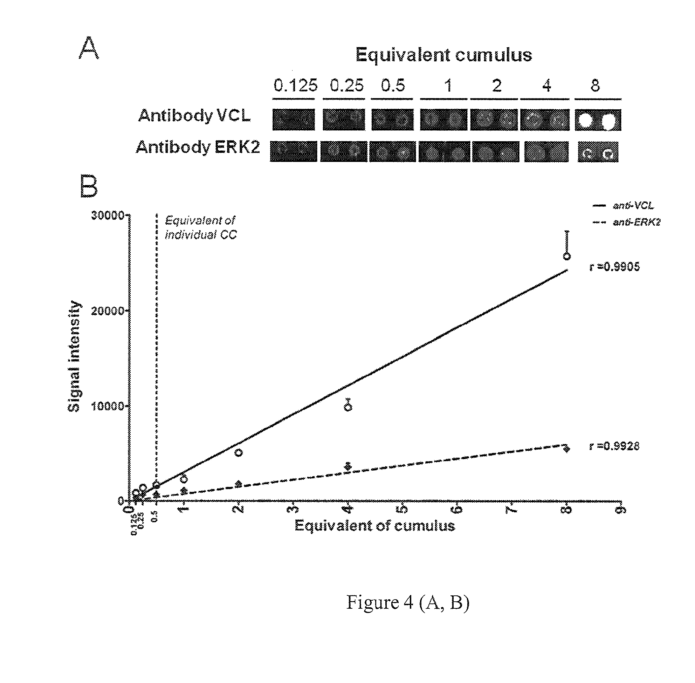

Figure 4 shows detection of VCL and ERK2 protein by Reverse Phase Protein Array on eight-fold dilution of a pool of 16 human cumulus cells. Signal intensities were expressed as mean of the two replicates. Linear regression was performed for VCL (solid line) and ERK2 (dotted line) and Pearson correlation was calculated.

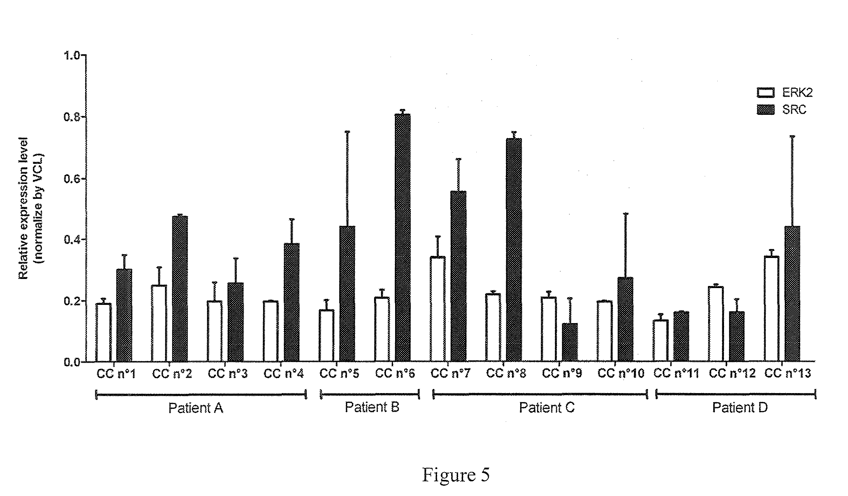

Figure 5 shows level of expression of ERK2 (white box) and SRC (black box), by Reverse Phase Protein Array on 13 individual cumulus cells from 4 patients (A to D). The equivalent of 0.5 (white box) and 0.25 (black box) individual cumulus cells were spotted in two replicates on array. Signal intensities were expressed as mean ± SD of the normalized signal of the replicates.

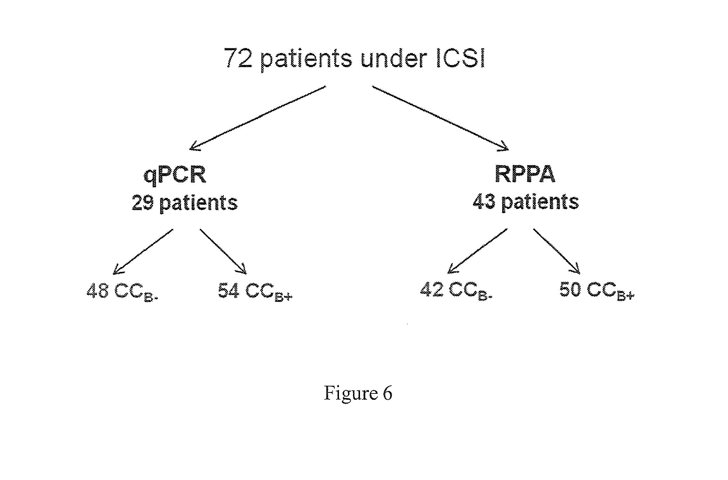

Figure 6 shows the distribution tree of patients included in the study. Patients were separated in two principals groups; microfluidic-based qPCR assay (qPCR) and Reverse Phase Protein Array (RPPA). qPCR group is composed of patients which have one CCB+ and at least one CCB-. CCB+, cumulus cells from mature oocyte yielding a blastocyst at day 5/6 of in vitro culture after fertilisation; CCB-, cumulus cells from mature oocyte which stopped developing before reaching the blastocyst stage after day 5/6 of in vitro culture after fertilisation.

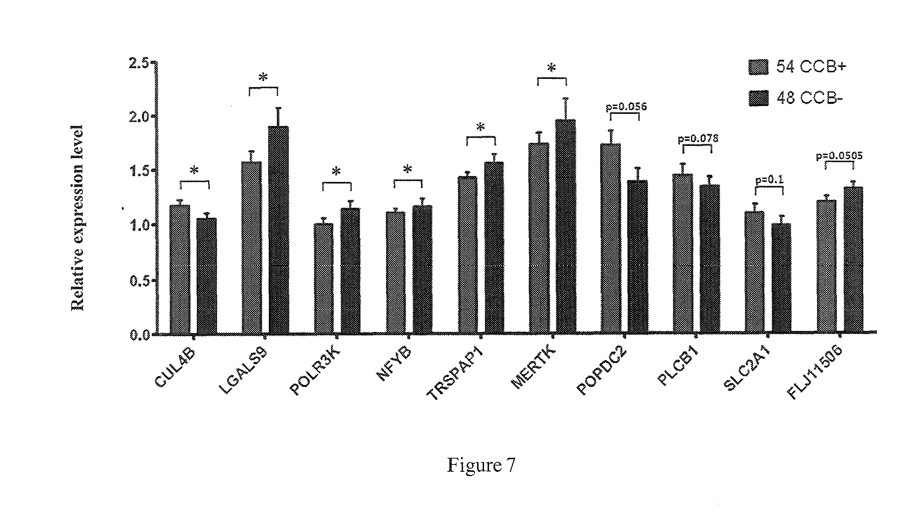

Figure 7 shows the relative expression level of the 10 genes most differentially expressed in cumulus cells according to oocyte developmental competence. Results were expressed as means ± SEM of relative expression to the reference genes RPL9 and RPL13. CCB+ (in grey), cumulus cells from mature oocyte yielding a blastocyst at day 5/6 of in vitro culture once fertilised; CCB- (in black), cumulus cells from mature oocyte which stopped developing at the embryo stage at day 5/6 of in vitro culture once fertilised; *, significant difference (p<0.05) with multifactor Anova.

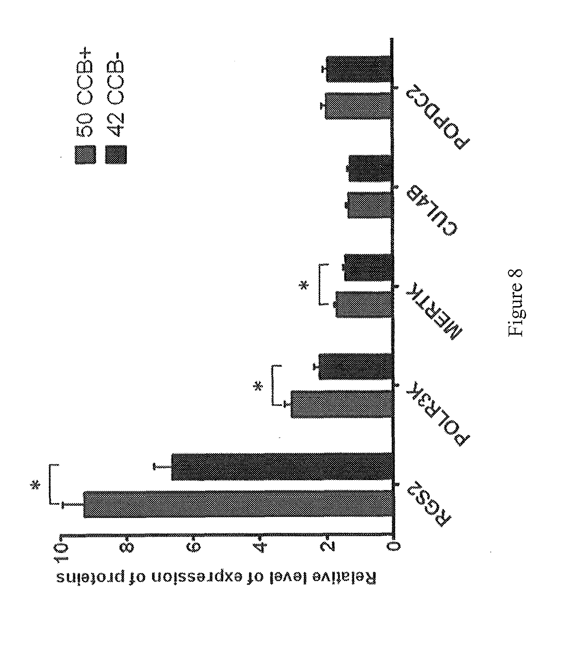

Figure 8 shows the relative expression level of protein in cumulus cells according to oocyte developmental competence. Results were expressed as means ± SEM of relative expression level to the reference protein VCL. CCB+ (in grey), cumulus cells from mature oocyte yielding a blastocyst at day 5/6 of in vitro culture once fertilised; CCB- (in black), cumulus cells from mature oocyte which stopped developing at the embryo stage at day 5/6 of in vitro culture once fertilised; *, significant difference (p<0.05) with one way Anova.

### EXAMPLES

### I. Example 1: Reverse phase protein array (RPPA) A new approach for the identification of cumulus cells biomarkers of oocyte quality

### Methods

### Cumulus cells recovery

Human cumulus cells (CCs) were collected from 6 patients undergoing intracytoplasmic sperm injection (ICSI) procedure for male infertility without taking into account the oocyte or embryo competence (Figure 1). Shortly before ICSI, human CCs were subjected individually to dissociation, as already described by Feuerstein et al., Gene expression in human cumulus cells: one approach to oocyte competence. Hum Reprod 2007, 22:3069-3077. Cumulus cells were separated from the oocyte with strippers after brief exposure to hyaluronidase (80 UI/ml, SynVitro HyadaseR, Medicult, Jyllinge, Denmark) at 37°C and were recovered individually or pooled together. Cumulus cells were washed in cold phosphate buffer saline (PBS). CCs from pooled cumulus were centrifuged at 300 g for 5 minutes whereas the individual cumulus were dissociated then equally distributed in 2 tubes before the centrifugation. The supernatants were removed and the pellets were stored at -80°C until RPPA assay. Only one tube corresponding to each individual CCs was used for RPPA assay which represent 0.5 equivalent CCs, the other was stored for future analysis. The ovarian stimulation protocol, the ICSI and the embryo culture procedures have been described in Guerif F. et al., Overnight incubation improves selection of frozen-thawed blastocysts for, Theriogenology 2003, 60:1457-1466, and embryo quality was not assessed.

### Cell culture and small interfering RNA transfection

HEK293 cells were grown in Minimum Essential Medium eagle (MEM) (Sigma-Aldrich, St Louis, MO, USA), supplemented with 10% FBS (Sigma-Aldrich) and antibiotics (100U/mL penicillin, 100 mg/mL streptomycin) (Sigma-Aldrich) at 37°C with 5% CO2 in a humidified atmosphere. The siRNA transfection was done according to the manufacturer's recommendations. Briefly, cells were seeded at a density of 2x104 120 cells/well in 6-well plates and were then cultivated for 24 h at 37 °C and 5 % CO2. Prior to transfection, complete medium was replaced with antibiotics-free medium.

Cells were transfected with 10µl of siRNA targeting VCL, RGS2, SRC or GAPDH (used as positive control), all purchased from Dharmacon (Lafayette, CO, USA). For each target, four individual siRNAs were pooled. Non-silencing siRNA control also purchased from Dharmacon. SiRNA were transfected by using 10 µl of Dharmafect (Dharmacon, Lafayette, CO, USA). Cells were harvested after 72 h of incubation.

### Cells lysis

The lysis buffer (MPER, Thermo Scientific) was supplemented with protease inhibitor (miniComplete, Roche) and phosphatase inhibitor (complete ultra 130 tablet, Roche).

HEK293 cells transfected with siRNA were trypsinized, pelleted, suspended in 50 µl of lysis buffer and lysed for 20 min at 4°C on a rotating wheel. After centrifugation, approximately 50 µl of protein extract was obtained. Individual and pooled CCs were suspended in 40 µl of lysis buffer and lysed for 20 min at 4°C on a rotating wheel. After centrifugation, approximately 40 µl of total protein extract was obtained.

### Western Blotting (WB)

An equal volume of 2X Laemmli buffer (32mM Tris pH 6.8, 8% SDS, 5% β-mercaptoethanol, 10% glycerol, 0.02% bromophenol blue) was added to HEK293 cell lysates, heated for 10 min at 95°C, resolved by 10% SDS-PAGE and transferred to nitrocellulose membrane (Protrans, Whatman, Maidstone, UK). Membranes were saturated with TBST-BSA (Tris 10mM, pH 8, 150mM NaCl with 0.1 % Tween 20 and 4% BSA) and probed with primary antibodies (see Table 2) in TBST-BSA over night at 4°C. Membranes were washed three times with TBST (Tris 10mM, pH 8, 150mM NaCl with 0.1 % Tween 20) before probed with secondary antibody (Peroxidase-conjugated F(ab')2 FragmentDonkey anti-rabbit IgG (1:2000) (Jackson ImmunoResearch Laboratories) or Peroxidase-conjugated F(ab')2 Fragment Goat anti-Mouse IgG (1:2000) (Jackson ImmunoResearch Laboratories)). WESTERN BLOT was revealed with Supersignal West Pico Chemiluminescent Substrate (Pierce Biotechnology, Rockford, IL, USA). Films (Amersham hyperfilm ECL) were scanned and the optical density of the signals was measured with ImageJ software (National Institutes of Health, USA).

**Table 2**

| **Antibody Name** | **Company** | **Species** | **Dilution RPPA** | **Dilution WB** |
|---|---|---|---|---|
| **ERK2** | Santa Cruz Biotechnology | Rabbit | 1:1000 | 1:10000 |
| Ref: sc-154 | | | | |
| **GAPDH** | Cell Signaling | Rabbit | 1:1000 | 1:10000 |
| Ref:5174 | | | | |
| **SRC** | Cell Signaling | Rabbit | 1:500 | 1:1000 |
| Ref:2109 | | | | |
| **VCL** | Sigma-Aldricht | Mouse | 1:1000 | 1:10000 |
| Ref:V9131 | | | | |
| **RGS2** | Abnova | Mouse | 1:500 | 1:5000 |
| Ref : H00005997-M01 | | | | |

### Reverse Phase Protein Array (RPPA)

Reverse Phase Protein Array design.

For each transfection a five-fold dilution of cells lysate were spotted in two replicates on the array. Total cells lysate of 16 individual CCs from 2 patients were pooled and used to determine the threshold of sensitivity of RPPA. Samples were spotted in two replicates using a 32-pin manual arrayer (Glass Slide Microarrayer, VP478, V&P Scientific, San Diego, CA, USA). According to the manufacturer's indications, [3-13] nL of sample were spotted on slide (Fast Slides, Whatman, Maidstone, UK) per array pin touch. An eight-fold dilution corresponding to a range of 16 to 0.125 equivalents CCs was used to be spotted on the array.
Total cells lysate of 13 individual CCs of 4 patients were analysed by RPPA and a two-fold dilution corresponding to a range of 0.5 to 0.25 equivalents CCs was used to be spotted in two replicates.

Reverse Phase Protein Array.

Antibodies targeting selected proteins were the same as those used for Western Blot. None of them were validated for RPPA prior to this study except anti-ERK2 (Dupuy L. et al., A highly sensitive near-infrared fluorescent detection method to analyze signalling pathways by reverse-phase protein array, Proteomics 2009, 9:5446-5454). Antibodies concentrations are listed in Table 2. RPPA were adapted from (Dupuy L., A highly sensitive near-infrared fluorescent detection method to analyze signalling pathways by reverse-phase protein array, Proteomics 2009, 9:5446-5454). Briefly, desiccated nitrocellulose-coated Fast-slides were printed with samples, using a 32-pin manual arrayer and desiccated again overnight. The immunodetection procedure was adapted from (Chan SM et al., Protein microarrays for multiplex analysis of signal transduction pathways, Nat Med 2004, 10:1390-1396). All antibodies were pre-cleared in FBS for 1 h at 37°C prior to use. After rehydration with PBS-Tween 20 0.1% (PBST), slides were blocked overnight at 4°C with 3% casein in PBST. Slides were probed with antibodies (listed in Table 2) 3h at room temperature (RT) in PBST with 20% FBS (PBST-FBS). Slides then were washed three times for 5min with three rinses with PBST between washes and then probed for 45min at RT with Peroxidase-conjugated F(ab')2 Fragment Donkey anti-rabbit IgG (1:2000) (Jackson ImmunoResearch Laboratories) or Peroxidase-conjugated F(ab')2 Fragment Goat anti-Mouse IgG (1:2000) (Jackson ImmunoResearch Laboratories) diluted in PBST-FBS. Slides were washed as previously described for signal amplification and slides were subsequently incubated for 10min at RT with BioRad Amplification Reagent (Amplified Opti-4CN Substrate Kit, BioRad, Hercules, CA, USA). Then, slides were rinsed six times, washed once for 5min in PBST with 20% DMSO (PBST-DMSO) and washed twice for 5min and rinsed in PBST as above. Slides were then probed for 1 h at RT with streptavidin AlexaFluors 680 conjugate (0.2 mg/mL) (AF680-streptavidin) (Invitrogen LifeTechnologies) in PBSTFBS. Finally, slides were air-dried by centrifugation at 2500 rpm for 15min and scanned at 700 nm with the Odyssey IR imaging system (LI-COR Biosciences, Lincoln, NE, USA) at a 42 µm resolution.

Scanned images of arrays were analysed with GenePix Pro6.0 software (Molecular Devices, Sunnyvale, CA, USA).

### Data analysis

Validation of antibodies.

For Western Blot, signal intensities were determined for each point with subtraction of minimum profile background. Signal intensity of the protein of interest was normalised by the signal of Vinculin (VCL), used as a protein of reference or by the signal of ERK2 (for the validation of anti-VCL) to calculate the relative expression level. Different exposure times were compared for each Western Blot experiment in order to avoid signal saturation. For RPPA mean value of intensity was determined for each spot with subtraction of the local background intensity. The mean value of intensity of the protein of interest was normalized by the mean value of intensity of Vinculin (VCL), used as a protein of reference or by the signal of ERK2 (for the validation of anti-VCL) to calculate the relative expression level. Pearson correlation analysis was performed between the dilution and the signal intensity (GraphPad Prism 5 Software, San Diego, CA, USA). Knockdown efficiency of the targeted proteins was estimated by comparing the signal intensity of targeted protein normalised by a reference protein (VCL and ERK2) in siRNA treated cells versus control siRNA treated cells (arbitrarily chosen as 100%).

Reverse Phase Protein Array on human cumulus cells.

The intensity of each spot was obtained after subtracting the local background intensity. Signal intensity is expressed for each sample as mean ± SD of the intensity of the two replicates. The mean value of intensity of ERK2 and SRC was normalized by the mean value of intensity of VCL (used as a protein of reference) to calculate the level of expression in each individual CCs.

### Results

### Validation of antibodies

Before their use in RPPA, the antibodies targeting the proteins of interest were assessed for their specificity by Western Blot on HEK293 cells lysates. A major band corresponding to the expected molecular weight was detected on each lane (Figure 2). Minor bands also appeared for the antibodies targeting SRC and VCL. It was further assessed the antibodies suitability for RPPA by measuring siRNA-mediated knock-down efficiency in HEK293 cells in parallel by Western Blot and RPPA from the same lysates. Pearson correlation analysis between the dilution and the signal intensity were greater than 0.9837. The knock-down efficiency quantified by RPPA was calculated with then dilution 1:2 and 1:4 corresponding to an unsaturated signal (Figure 3A). The extinction level of VCL, GAPDH and SRC were acceptable in Western Blot and in RPPA with difference in knockdown efficiencies measured by Western Blot and RPPA for SRC and GAPDH (Figure 3C) which validated these antibodies for RPPA. However, the knockdown efficiency of RGS2 measured in RPPA was too low (13% compared to 66% measured by Western Blot) to allow RGS2 antibody validation.

Therefore, the antibodies targeting VCL, ERK2 and SRC were used for the detection of the proteins in human CCs.

### Reverse Phase Protein Array on human cumulus cells

Defining sensitivity and threshold of RPPA on a pool of 16 human cumulus cells.

The sensitivity of RPPA was evaluated on a pool of 16 human cumulus by assessing the signal observed with the validated antibodies using serial dilutions of the pool while the lowest level of detection was determined.

Signal intensity was saturated with all antibodies at the highest concentration (equivalent of 16 CCs spotted). Signals intensities were clearly correlated with the dilution of cells from the pool of the 16 cumulus with r=0.9905 for VCL and r=0.9928 for ERK2 (p<0.0001) (Figure 4), r=0.9589 for SRC (p=0.0006) and r=0.9284 for GAPDH (p=0.02).

Additionally, a signal was detected for the dilution corresponding to 0.25 equivalent cumulus spotted for all antibodies. After taking into account the volume printed per spot and estimating the number of cells composing a cumulus, the equivalent of 0.5 to 2 cells were printed per spot. Signals corresponding to the three antibodies were above the detection threshold.

Detection of proteins of interest in individual human cumulus cells.

The detection of protein SRC, ERK2 and VCL has then been carried out on 13 individual CCs. Signals were detected for each sample of individual CCs (*i.e.* 0.5 equivalent of individual CCs) for the 3 proteins and remained detectable at 0.25 equivalent of individual CCs in all 13 cumulus analysed. Moreover, the intensity of signals of the three proteins was significantly correlated (p<0.001) between the two dilutions corresponding to the equivalent of 0.5 CCs and 0.25 equivalent of CCs.

The level of expression of ERK2 was almost the same across the 13 individual CCs while the level of expression of SRC was different between the 13 individual CCs of the 4 patients and between the CCs from a same patient (Figure 5).

### Conclusion

In this study it was demonstrated that RPPA combining near-infrared (NIR) dyes can be applied to human cumulus cells to measure potential biomarkers of oocyte competence. ERK2 proteins were detectable in individual human cumulus. Moreover, after validation by siRNA approach in HEK293 cells the antibodies targeting VCL and SRC, it was possible to detect those two proteins of interest with the same sensitivity.

Defining embryo quality with non-invasive methods continues to be the major goal in ART while morphological criteria of early embryo development to predict further development or implantation remain poorly predictive. Various approaches involving transcriptomics, proteomics and metabolomics on the embryo or its cellular or non-cellular environment were proposed. The cumulus cells surrounding the oocyte may be targeted to develop non-invasive method to investigate the oocyte developmental competence. While the proteins remains the final effectors in the cells, no link between the product of these mRNAs (*i.e.* proteins) and the oocyte competence has been established to date. This is due, in large part, to a lack of technology sensitive enough to analyse specific proteins from the minute amount of cumulus cells available.

Protein microarray-based methods, particularly RPPA are very sensitive and allow the detection of multiples specific targeted proteins in small quantities of biological material compared to other proteome approach such as Western Blot and mass spectrometry which conduct respectively to follow limited targeted proteins or global proteome from a same sample. Moreover, RPPA was successfully applied to biomarker profiling in cancer diseases (see Mueller C. et al., Reverse phase protein microarrays advance 380 to use in clinicalTrials, Mol Oncol 2010, 4:461-481). In this context, the Inventors thought that RPPA could be used to evaluate the level of protein expression of some potential biomarkers of oocyte developmental competence in cumulus cell. In order to achieve a proof of concept, the Inventors used two potential biomarkers RGS2 and SRC (data not shown) and a housekeeping protein VCL used as reference protein.

As a first step, the Inventors used siRNA targeting the proteins of interest in HEK293 cells as a mean to validate the antibodies for their use in RPPA. Using Western blotting, the knock-down efficiency for GAPDH, used as positive control of the transfection, was estimated at 39%. The silencing of transcription was confirmed by the knock-down efficiencies of VCL, RGS2 and SRC reached (56.5%, 66% and 46% respectively) of the control. The knockdown efficiencies estimated by RPPA were close to those observed by Western blot for SRC, VCL and GAPDH, thus allowed us to validate these antibodies for RPPA.

RPPA sensitivity was assessed on pooled then individual human CCs using the validated antibodies. The study by Dupuy *et al.* 2009 (Dupuy L. et al., A highly sensitive near-infrared fluorescent detection method to analyze signalling pathways by reverse-phase protein array. Proteomics 2009, 9:5446-5454) showed that RPPA combining near-infrared (NIR) dyes allow the detection of phosphorylated ERK in the equivalent of two HEK293 cells printed per spot. In line with this work, the Inventors showed that ERK2 protein can be detected for a dilution corresponding to less than one cell of an individual human cumulus printed per spot. Furthermore, VCL and SRC proteins were detected with the same range of sensitivity.

Even though antibody is a challenging problem, RPPA appears to be a useful technology for biomarkers detection for clinical diagnostic in individual cumulus cells (sensitive, high throughput, cost and material effective) compared to Western Blot, 2D electrophoresis and mass spectrometry which were already tested on human cumulus cells. The siRNA approach has shown its efficiency for antibody validation (Mannsperger HA et al., RNAi-based validation of antibodies for reverse phase protein arrays, Proteome Science 2010, 8:69 ; Hennessy BT et al., A Technical Assessment of the Utility of Reverse Phase Protein Arrays for the Study of the Functional Proteome in Non-microdissected Human Breast Cancers, Clinical Proteomics 2010, 6:129-151). However, this approach is limited by the protein characteristics (*i.e.* turn-over of synthesis, half-life and cellular function) and requires specific development for each target. In this context, siRNA is not optimal for rapid and high throughput validation of a large collection of antibodies. Alternatively, overexpression of the targeted protein by plasmid transfection might be a better alternative for future antibody validations.

### II. Example 2: Identification of the most differentially expressed genes according to oocyte developmental competence.

### Materials and Methods

### Patient selection and IVF treatment

Seventy two patients were included in this study, all undergoing intracytoplasmic sperm injection (ICSI) procedure for male infertility. A group of twenty nine patients was selected for the qPCR analysis on the basis that at least one embryo had reached the blastocyst stage while at least one embryo arrested its development after 6 days of culture (Figure 6). The ovarian stimulation protocol, the ICSI and the embryo culture procedures have been described by Guerif F. et al., 2003.

### Cumulus cell recovery and assessment of oocyte and embryo quality

Individual CCs were collected as described in Feuerstein P. et al., 2012.

Assessment of oocyte and embryo quality was described in Feuerstein P. et al., 2012. CCs from an oocyte yielding a blastocyst after 5/6 days of in vitro culture were denominated CC_{B+} and CCs from an oocyte arresting development at the embryo stage after 5/6 days of in vitro culture were denominated CC_{B-}.

### Microfluidic-based gPCR assay

The following procedures were used in order to comply as far as possible with the Minimum Information for Publication of Quantitative PCR experiments MIQE guidelines (Bustin SA. et al., (2009) The MIQE Guidelines: Minimum Information for Publication of Quantitative Real-Time PCR Experiments, Clinical Chemistry 55: 611-622).

### RNA extraction and cDNA synthesis

Total RNA extraction and removal of genomic DNA were performed using the RNeasy^{®} Micro Kit (Qiagen, Courtaboeuf, France) according to the manufacturer's recommendations. The quality and integrity of RNA samples were assessed using the 2100 Bioanalyser and RNA 6000 Nano LabChip kit series II (Agilent Technologies). Total RNA was quantified using a Nanodrop^{®} ND-1000 spectrophotometer (Nyxor Biotech, Paris, France). Only RNA samples that displayed a RIN (RNA integrity number) greater than or equal to 7 were reverse transcribed to cDNA. The mean quantity of RNA per cumulus was 99 ng (range 21-205 ng). Total RNA from each sample was reverse transcribed into cDNA using the iScript™ cDNA Synthesis kit (Bio-Rad Laboratories, Marnes-la-Coquette, France) with a blend of oligo(dT) and random hexamer primers to provide complete RNA sequence representation.

### Gene selection, primer design and qPCR design

Among 96 genes selected, 95 were the most differentially expressed transcripts of the microarray study (GSE37277) and *RPL19* was selected as reference gene. All primers were designed by DELTAgene Assays with wet validation (Fluidigm Europe B.V., Amsterdam, Netherlands). Description of the primers used for microfluidic-based qPCR assay is given in Table 1 above. The level of gene expression was assessed over cumulus cells from 102 mature oocytes, including 54 CC_{B+} and 48 CC_{B-}.

### Microfluidic-based qPCR assay

qPCRs were performed on Biomark HD (Fluidigm Europe B.V.), in 2 microfluidic multiplex 96.96 dynamic array chip according to the Genotoul Protocol. Briefly, a 14-cycle pre-amplification reaction was performed for each sample in 5 µl by pooling 1.25 µl of all primer pairs (each primer at a concentration of 20 nM), 1.3 µl of cDNA and 2.5 µl of TaqMan® PreAmp Master Mix Kit (Applied Biosystems, 4391128). Pre-amplification was performed with the following thermal cycling conditions: initial activation at 95°C for 10 minutes, followed by 14 cycles of 15 s at 95°C and 4 min at 60°C. All samples of pre-amplified cDNA was diluted by 5-fold in TE-buffer 1X (Dutscher, 091575). Sample Mix contained 440 µl of 2X TaqMan Gene Expression Master Mix (Applied Biosystems, 4369510), 44 µl of 20X DNA Binding Dye Sample Loading Reagent (Fluidigm, 100-0388), 44 µl of 20X EvaGreen (Interchim, BI1790) and 132 µl of TE buffer 1X. For each sample 6 µL of Sample Pre-Mix was added to 2 µl of pre-amplified cDNA 1/5 diluted and 5 µl was loaded in Sample Inlets on the chip. For each individual assay, 4 µl of 2X Assay Loading Reagent (Fluidigm, 85000736), 2 µl of TE Buffer 1X and 2 µl of primer (20 nm) were pooled and 5 µl were loaded into one of the Assay Inlets on the chip. The Biomark's cycling program (10min Hot Start, 35 cyc) with a final melting was used. Pooled human cumulus cells were used to establish the standard curve by 4-fold serial dilutions in TE buffer 1X and repeated for each array chip. A no template control (NTC) and a positive control were included in all assays.

### Data analysis

For each gene, the threshold of Cq was defined for the 2 array chips on the first point of the standard curve. All gene analysis with a variation between array chip of the positive control or the first point of the standard curve greater than 0.5 were discarded (see supplemental data). The presence of multiplex on the melting curve was another criterion to discard the data point. Among 96 genes, 74 fulfilled all the criteria. *RPL19* and *RPL13* were selected by the GeNorm algorithm (Vandesompele J. et al., (2002) Accurate normalization of real-time quantitative RT-PCR data by geometric averaging of multiple internal control genes, *Genome Biology* 3: RESEARCH0034) as the most stables genes. Thus, the data were normalized to the mean of *RPL19* and *RPL13* relative concentrations.

Statistical analysis of results was performed using multifactor Anova (phenotype [i.e. CC_{B+} vs. CC_{B-}] and patient [n=29] as variables) with statistical significance defined as p<0.05 with Statview software (SAS Institute Inc.).

### Results

Among 95 genes that were selected for microfluidic-based qPCR assay, 72 genes were analysed by a multifactor Anova. *CUL4B (cullin 4B)* was up regulated in CC_{B+} compared to CC_{B-} with statistical significance and five genes *[LGALS9 (lectin galactoside-binding soluble 9), POLR3K (polymerase (RNA) III (DNA directed) polypeptide K), NFYβ (nuclear transcription,factor Y beta), TRSPAP1 (also known as TRNAU1AP; tRNA selenocysteine 1 associated protein 1) and MERTK (c-mer proto-oncogene tyrosine kinase*)] were down regulated in CC_{B+} compared to CC_{B-} with statistical significance (Fig. 7). In addition, four genes (i.e.: *POPDC2, PLCB1, SLC2A1 and FLJ11506*) displayed differences that were just below statistical significance. The same expression profile was observed in qPCR and microarray for *CUL4B, LGALS9, POLR3K, PLCB1, POPDC2* and *SLC2A1.*

### III. Example 3: Impact of developmental competence and the patient variability respectively on the level of gene expression.

### Data analysis

In order to evaluate the impact of developmental competence and the patient variability respectively on the level of gene expression, an analysis of variance (Anova) with the Generalised Linear Mixed Model (GLMM) procedure was performed for each gene with Statistical Analysis System (SAS®) software. The model chosen was fitted for each gene independently yijn=µ+Pi+Aj+PiAj+εijn, where yijn is the gene expression level in the nth CC for a gene according to the ith phenotype from the jth patient, µ is the gene expression level mean, Pi the fixed effect of the ith phenotype (i= CC_{B+} or CC_{B-}), Aj the random effect of the jth patient (j=1 to 29), (PA)ij the first-order interaction between variables phenotype and patient, and εijn the residual random effect. The levels of significance of the model and the different effects were set at p<0.01 and p<0.05, respectively.

### Results

### Biomarkers of oocyte developmental development

### Assessment of the respective impact of developmental competence and patient variability on the level of gene expression.

In order to further validate the 10 genes identified as potential biomarkers of oocyte developmental competence, the impact of patients on the level of gene expression was assessed according to oocyte developmental competence. Using a Generalised Linear Mixed Model, the Inventors found the statistical significance of the model for all the genes. The level of expression of *POLR3K* was related to oocyte developmental competence without any other interaction. Despite a significant patient effect, the level of expression of *CUL4B* remained clearly related to oocyte developmental competence. Moreover the levels of expression of the other genes were unrelated to oocyte developmental competence (Table 3).

**Table 3. Test of hypotheses for Mixed Model Analysis of Variance, impact of developmental competence and patient variability of the level of gene expression.**

| **Effects** / **yijn (Gene)** | **P*****i** (Phenotype)* | **A*****j** (Patient)* | **(PA)*****ij** (Patient*Phenotype)* |
|---|---|---|---|
| ***CUL4B*** | 0.0410* | <0.0001* | 0.3547 |
| ***POLR3K*** | 0.0301* | 0.2902 | 0.2999 |
| ***LGALS9*** | 0.0673 | 0.8976 | 0.0575 |
| ***FLJ11506*** | 0.0898 | 0.0009* | 0.1690 |
| ***PLCB1*** | 0.2204 | 0.0185* | 0.0079* |
| ***TRSPAP1*** | 0.1088 | 0.3079 | 0.0046* |
| ***NFYB*** | 0.1888 | 0.0063* | 0.0021* |
| ***POPDC2*** | 0.1376 | 0.0996 | 0.0444* |
| ***SLC2A1*** | 0.1864 | 0.0192* | 0.0668 |
| ***MERTK*** | 0.2604 | 0.0185* * | <.0001* |
| yijn gene expression level in nth cumulus cells for a gene according to the ith phenotype from jth patient; µ gene expression level mean; Pi fixed effect of ith phenotype (i= CC_{B+} cumulus cells from mature oocyte yielding a blastocyst at day 5/6 of in vitro culture once fertilised and i= CC_{B-} cumulus cells from mature oocyte which stopped developing at the embryo stage at day 5/6 of in vitro culture once fertilised); Aj random effect of the jth patient (j=1 to 29); (PA)ij first-order interaction between variables phenotype and patient; *, Statistical significance of model factors for the levels of expression of the five genes (p<0.05) | | | |

### Discussion

Indeed, while studies identified potential biomarkers using different set of samples and different techniques for transcriptomic approaches, few reports have focused on the inter- and intra-patient variability to really ensure that biomarkers are correlated to oocyte quality. Adriaenssens et al., 2011 showed that genes identified in CCs as potential biomarker of oocyte quality were associated with patient and treatment characteristics and used multivariable models based on CC gene expression to predict embryo development and pregnancy. The model of Anova test with GLMM of the invention allowed the Inventors to take into account the inter- and inter-patient variability as one variable, named "patient variability" and evaluate the respective influence of developmental competence and patient variability on the level of gene expression. It was studied an independent set of 29 patients with at least one blastocyst and one arrested embryo per patient. Among the 10 candidate genes previously identified, only 2 of them (i.e. *POLR3K* and *CUL4B)* remained related to oocyte developmental competence, independently of patient variability.

### IV. Example 4: Identification of most differentially expressed proteins according to oocyte developmental competence.

### Materials and Methods

### Patient selection and IVF treatment

As indicated for example 2, seventy two patients were included in this study, all undergoing intracytoplasmic sperm injection (ICSI) procedure for male infertility. Forty three patients were included in RPPA analysis (Figure 6). The ovarian stimulation protocol, the ICSI and the embryo culture procedures have been described by Guerif F, et al., 2003.

### Cumulus cell recovery and assessment of oocyte and embryo quality

Individual cumulus cells (CCs) were washed in cold phosphate buffer saline (80 IU/ml, SynVitro Hyadase, Medicult, Jyllinge, Denmark). Cumulus were dissociated as described in Feurestein et al., equally divided in 2 tubes and then centrifuged at 300 g for 5 minutes. The supernatants were removed and for one tube the pellet was stored at -80°C until potential RPPA assay and for the other tube the pellet was re-suspended in 50 µl of RLT buffer of the RNeasy© Micro Kit (Qiagen, Courtaboeuf, France) before storage at -80°C.

### Assessment of oocyte and embryo quality

Assessment of oocyte and embryo quality was described in Feuerstein *et al.,* 2012. CCs from an oocyte yielding a blastocyst after 5/6 days of in vitro culture were denominated CC_{B+} and CCs from an oocyte arresting development at the embryo stage after 5/6 days of in vitro culture were denominated CC_{B-}.

### Reverse Phase Protein Array (RPPA)

### Cumulus cells lysis

The lysis buffer (MPER, Thermo Scientific) was supplemented with protease inhibitor (miniComplete, Roche) and phosphatise inhibitor (complete ultra tablet, Roche). CCs were suspended in 40 µl of lysis buffer and lysed for 20 min at 4°C on a rotating wheel. After centrifugation, approximately 40 µl of protein extract was obtained.

### Antibodies selection

Antibodies targeting selected proteins were chosen on The Human Protein Atlas (Uhlen M, Oksvold P, Fagerberg L, Lundberg E, Jonasson K, et al. (2010) Towards a knowledge-based Human Protein Atlas. Nat Biotech 28: 1248-1250) when they were validated for protein array (Table 4). Anti-VCL was validated by siRNA approach and VCL was used as reference protein.

**Table 4. List of antibody used for RPPA.**

| **Antibody Name** | **Company** | **Species** | **Dilution** |
|---|---|---|---|
| **POPDC2 (ref: HPA024255)** | Sigma-Aldricht | Rabbit | 1:1000 |
| **MERTK (ref: HPA036196)** | Sigma-Aldricht | Rabbit | 1:1000 |
| **POLR3K (ref: HPA005891)** | Sigma-Aldricht | Rabbit | 1:1000 |
| **CUL4B (ref: HPA011880)** | Sigma-Aldricht | Rabbit | 1:1000 |
| **VCL (ref:V9131)** | Sigma-Aldricht | Mouse | 1:1000 |
| **RGS2 (ref:157H00005997-B01P)** | Abnova | Mouse | 1:1000 |

### Reverse Phase Protein Array design

Cells from 92 individual cumulus from mature oocytes were analysed by RPPA (representing a mean of 14500 cells per cumulus), including 52 CC_{B+} and 40 CC_{B-}. A two-fold dilution corresponding to a range of equivalent of 0.5 individual CC to 0.25 individual CC were also spotted in two replicates as described in example 2.

### Reverse Phase Protein Array

RPPA were adapted from Dupuy L. et al., 2009. Briefly, desiccated nitrocellulose-coated slides (Fast Slides, Whatman, Maidstone, UK) were printed with samples, using a 32-pin manual arrayer (Glass Slide Microarrayer, VP478, V&P Scientific, San Diego, CA, USA) and desiccated again overnight. According to the manufacturer's indications, 3-13 nL of sample were spotted on Fast-slide per array pin touch. The immunodetection procedure was adapted from Chan SM et al, 2004. All antibodies were pre-cleared in FBS for 1 h at 37°C prior to use. After rehydration with PBS-Tween 20 0.1% (PBST), slides were blocked overnight at 4°C with 3% casein in PBST. Slides were probed with antibodies (listed in table 4) 3h at room temperature (RT) in PBST with 20% FBS (PBST-FBS). Slides then were washed three times for 5min with three rinses with PBST between washes and then probed for 45min at RT with Peroxidase-conjugated F(ab')2 Fragment Donkey anti-rabbit IgG (1:2000) (Jackson ImmunoResearch Laboratories ref: 711-036-152) or Peroxidase-conjugated F(ab')2 Fragment Goat anti-Mouse IgG (1:2000) (Jackson ImmunoResearch Laboratories ref: 115-036-072) diluted in PBST-FBS. Slides were washed as previously described for signal amplification and slides were subsequently incubated for 10min at RT with BioRad Amplification Reagent (Amplified Opti-4CN Substrate Kit, BioRad, Hercules, CA, USA). Then, slides were rinsed six times, washed once for 5min in PBST with 20% DMSO (PBST-DMSO) and washed twice for 5min and rinsed in PBST as above. Slides were then probed for 1 h at RT with streptavidin AlexaFluors 680 conjugate (0.2 mg/mL) (AF680-streptavidin) (Invitrogen LifeTechnologies) in PBST-FBS. Finally, slides were air-dried by centrifugation at 2500 rpm for 15min and scanned at 700 nm with the Odyssey IR imaging system (LI-COR Biosciences, Lincoln, NE, USA) at a 42 µm resolution.

### Data analysis

Scanned images of arrays were analysed with GenePix Pro6.0 software (Molecular Devices, Sunnyvale, CA, USA). The mean value of intensity was determined for each spot with subtraction of the local background intensity. The mean value of signals was normalized according to the mean value of signals of the reference protein *(i.e.* VCL). The outliers corresponding to the maximum and the minimum values in each group were deleted for the statistical analysis of RPPA results. One way variance analysis was used with statistical significance defined as p<0.05 with Statview software (SAS Institute Inc.).

### Reverse Phase Protein Array

Individual cumulus cells (CCs) were washed in cold phosphate buffer saline (80 IU/ml, SynVitro Hyadase, Medicult, Jyllinge, Denmark). Cumulus were dissociated as described in Feuerstein et al., 2012, equally divided in 2 tubes and then centrifuged at 300 g for 5 minutes. The supernatants were removed and for one tube the pellet was stored at -80°C until potential RPPA assay and for the other tube the pellet was re-suspended in 50 µl of RLT buffer of the RNeasy® Micro Kit (Qiagen, Courtaboeuf, France) before storage at -80°C.

### Assessment of oocyte and embryo quality

Assessment of oocyte and embryo quality was described in Feuerstein et al, 2012. CCs from an oocyte yielding a blastocyst after 5/6 days of in vitro culture were denominated CC_{B+} and CCs from an oocyte arresting development at the embryo stage after 5/6 days of in vitro culture were denominated CC_{B-}.

### Results

*CUL4B* and *POLR3K, MERTK* and *POPDC2,* identified as biomarkers of oocyte developmental competence at mRNA level were selected to being assessed as protein biomarker in 92 individuals cumulus by RPPA. The gene *RGS2* was identified as a biomarker in our previous work and was also selected. The level of expression of the proteins RGS2, POLR3K and MERTK was significantly increased in CC_{B+} compared to CC_{B-} (Fig. 8). No difference was observed between CC_{B+} and CC_{B-} on the level of protein expression of CUL4B and POPDC2.

### Conclusions

Among 95 genes differentially expressed according the oocyte developmental competence by microarray, the Inventors showed that the genes *CUL4B, LGALS9, POLR3K, NFYβ, TRSPAP1 and MERTK* were differentially expressed with a statistical difference. Interestingly, they also demonstrated that the proteins RGS2, POLR3K and MERTK were differentially expressed according the oocyte developmental competence.

Their strategy was based on two stages: 1) identify new potential biomarkers of oocyte developmental competence from results of a transcriptomic approach on individual CCs by high throughput qPCR; 2) evaluated the level of expression of the proteins coded by some of these genes in regard to oocyte quality.

Most of the recent studies focused on the somatic cells surrounding the oocyte which identified through microarray approach potential biomarkers of oocytes quality validated their results by qPCR (Assou S. et al.; Human cumulus cells as biomarkers for embryo and pregnancy outcomes, Molecular Human Reproduction 2010, 16:531-538). However, these two approaches have different output and while microarray can highlight a large number of differentially expressed genes, the validation by qPCR have to focus on a part of them. The Inventors used BiomarkHD system to extend the potential biomarkers list by assessing 95 genes of the 308 differentially expressed genes according oocyte developmental competence identified by microarray in a previous study (Feuerstein et al, 2012). Moreover, they used independent set of samples to ensure that the expression profile was really correlated with the situations being compared and was not sample dependent (van Montfoort AP et al. (2008) Differential gene expression in cumulus cells as a prognostic indicator of embryo viability: a microarray analysis. Molecular Human Reproduction 14: 157-168). This approach led to identified "only" 10 new potentials biomarkers over the 94 selected genes. This apparent 'loss' of differentially expressed genes may partly be attributed to technical differences between microarray and qPCR approaches (Morey J, et al. (2006) Microarray validation: factors influencing correlation between oligonucleotide microarrays and real-time PCR. Biological Procedures Online 8: 175-193) and partly be the result of inter-patient variation due to the utilization of independent set of samples between the two approaches.

Transcriptomic analyses do not warrant information about the ultimate effectors in cells which remain based on protein-protein interactions or cellular signalling pathways. However, few studies addressed on the CCs proteome according to the oocyte quality mainly because of a lack of sensitivity of the technics. Recently, 2D electrophoresis was used to analysed the proteome of pooled CCs according ovarian protocol stimulation [Hamamah] and MALDI-TOF MS was used to study the lipid profile of cumulus cells and potentially be used as a supporting tool to predict pregnancy (Montani D, et al. (2012) The follicular microenviroment as a predictor of pregnancy: MALDI-TOF MS lipid profile in cumulus cells. Journal of Assisted Reproduction and Genetics 29: 1289-1297).Thereby, RPPA was used to detect and analyse potential biomarkers related to oocyte developmental competence through a non-invasive procedure involving individual cumulus cells in human.

Although that mRNA abundance does not always correlate with the level of the corresponding proteins (Shankavaram UT, et al. (2007) Transcript and protein expression profiles of the NCI-60 cancer cell panel: an integromic microarray study. Molecular Cancer Therapeutics 6: 820-832.), it was selected targeted proteins among the potential biomarkers which were identified at mRNA level. Among the five proteins selected three *(i.e.* RGS2, POLR3K and MERTK) were differentially expressed according the oocyte developmental competence.Interestingly, the level of gene expression of *MERTK* was lower in the CC_{B+} compared to the CC_{B-} while it was inverse at the protein level. Such discrepancy might be explained by differences in mRNA stability, degradation/synthesis rates or post-translational modification of the proteins.

In conclusion, the Inventors have surprisingly identified new biomarkers at mRNA level in CCs. And three of these proteins were validated as potential biomarker of the oocyte developmental competence.

## Claims

1. An *in vitro* method for evaluating developmental competence of an oocyte or an embryo comprising the step of assessing the expression level of at least one ovarian marker in a sample by:
a) assessing the expression level of at least one gene chosen in the group consisting of: *CUL4B, LGALS9, POLR3K, NFYβ, TRSPAP1, MERTK, POPDC2, PLCB1, SLC2A1 and FLJ11506,* preferably in the group consisting of: *CUL4B, LGALS9, POLR3K, NFYβ, TRSPAP1 and MERTK,* and more preferably in the group consisting of: *CUL4B and POLR3K* ; or
b) assessing the expression level of at least one protein chosen in the group consisting of: RGS2, POLR3K and MERTK, preferably chosen in the group consisting of: POLR3K and MERTK.

2. The method according to claim 1 wherein said ovarian marker is a cumulus cell marker.

3. The method according to any one of claims 1 or 2, wherein said oocyte is a mammalian oocyte and said embryo is a mammalian embryo.

4. The method according to any one of claims 1 to 3, wherein said oocyte is a human oocyte and said embryo is a human embryo.

5. The method according to any one of claims 1 to 4, further comprising the step of comparing the expression level of said at least one ovarian marker with a control expression level.

6. The method according to any one of claims 1 to 5, wherein the expression level of said at least one ovarian marker is assessed by determining the expression level of mRNA transcripts or mRNA precursors of at least one gene chosen in the group consisting of: *CUL4B, LGALS9, POLR3K, NFYβ, TRSPAP1, MERTK, POPDC2, PLCB1, SLC2A1 and FLJ11506* and combination thereof, preferably chosen in the group consisting of: *CUL4B* and *POLR3K.*

7. The method according to any of claims 1 to 6, wherein the expression level of mRNA transcripts or mRNA precursors is assessed by real-time quantitative polymerase chain reaction.

8. The method according to any one of claims 1 to 5, wherein the expression level of said at least one ovarian marker is assessed by determining the expression level of at least a protein chosen in the group consisting of: RGS2, POLR3K and MERTK, preferably in the group consisting of: POLR3K and MERTK.

9. The method according to any of claims 1 to 5 and 8, wherein the expression level of at least a protein is assessed by Reverse phase protein array.

10. The method according to any one of the previous claims, comprising a step of assessing the level expression of at least 2, 3, 4, 5, 6, 7, 8, 9, 10 or 11 ovarian markers chosen in the group consisting of: *CUL4B, LGALS9, POLR3K, NFYβ, TRSPAP1, MERTK, RGS2, POPDC2, PLCB1, SLC2A1 and FLJ11506,* preferably in the group consisting of: *LGALS9, POLR3K, NFYβ, TRSPAP1, R GS2 and MERTK.*

11. The method according to any one of the previous claims, comprising the steps of:
a) assessing the expression level of at least 2, 3, 4, 5, 6, 7, 8, 9, 10 or 11 genes chosen in the group consisting of: *CUL4B, LGALS9, POLR3K, NFYβ, TRSPAP1, MERTK, RGS2, POPDC2, PLCB1, SLC2A1 and FLJ11506*; and
b) assessing the expression level of at least 2 or 3 proteins chosen in the group consisting of: RGS2, POLR3K and MERTK.

12. A method of assisted reproduction, comprising a step of evaluating developmental competence of the oocyte as defined in any one of the previous claims.

13. A method of oocyte or embryo conservation, comprising a step of evaluating developmental competence of said oocyte or embryo as defined in any one of the previous claims 1 to 11.

14. A method for selecting embryo to be transferred to a subject, comprising a step of evaluating developmental competence of the oocyte giving rise to said embryo or of said embryo, said step being as defined in any one of the previous claims 1 to 11.

15. A method to evaluate the efficiency of a treatment of a patient comprising the step of assessing the expression level of at least one ovarian marker as defined in any one of the previous claims 1 to 11.

16. *In vitro* method for screening compounds capable to stimulate or to inhibit to oocyte competence, comprising the steps of:
a) contacting at least one cumulus cell with a compound to be screened for activity to stimulate or inhibit the competence of an oocyte; and
b) determining an expression level of at least one ovarian marker in said at least one cumulus cell contacted with said compound, wherein said at least one follicular cell marker is selected from the group consisting of: *CUL4B, LGALS9, POLR3K, NFYβ, TRSPAP1, MERTK, RGS2, POPDC2, PLCB1, SLC2A1 and FLJ11506* and combinations thereof, preferably in the group consisting in *CUL4B, LGALS9, POLR3K, NFYβ, TRSPAP1 and MERTK* and combinations thereof.

17. Use of the expression level of at least one ovarian marker chosen in the group consisting of: : *CUL4B, LGALS9, POLR3K, NFYβ, TRSPAP1, MERTKPOPDC2, PLCB1, SLC2A1 and FLJ11506* and combinations thereof, preferably at least one, preferably at least 2, at least 3, at least 4, at least 5 or 6 genes chosen in the group consisting *of: : CUL4B, LGALS9, POLR3K, NFYβ, TRSPAP1 and MERTK,* for evaluating developmental competence of a oocyte and combinations thereof.

18. Kit for evaluating developmental competence of a oocyte comprising means for determining the expression level of at least one, preferably at least 2, at least 3, at least 4, at least 5, at least 6, at least 7, at least 8, at least 9 or 10 ovarian markers chosen in the group consisting of: *CUL4B, LGALS9, POLR3K, NFYβ, TRSPAP1, MERTK, POPDC2, PLCB1, SLC2A1 and FLJ11506* and combinations thereof.


SEQUENCE LISTING

<110>  INSTITUT NATIONAL DE LA RECHERCHE AGRONOMIQUE
       UNIVERSITE FRANCOIS RABELAIS DE TOURS
       CENTRE NATIONAL DE LA RECHERCHE SCIENTIFIQUE (CNRS)

<120>  METHOD FOR EVALUATING DEVELOPMENTAL COMPETENCE OF AN OOCYTE

<130>  70733D32622

<160>  37

<170>  PatentIn version 3.5

<210>  1
<211>  3632
<212>  DNA
<213>  Homo sapiens


<220>
<221>  misc_feature
<223>  c-mer proto-oncogene tyrosine kinase (MERTK)

<400>  1
actcactgcc cgggccgccc ggacagggag cttcgctggc gcgcttggcc ggcgacagga      60

caggttcggg acgtccatct gtccatccgt ccggagagaa attacagatc cgcagccccg     120

ggatggggcc ggccccgctg ccgctgctgc tgggcctctt cctccccgcg ctctggcgta     180

gagctatcac tgaggcaagg gaagaagcca agccttaccc gctattcccg ggaccttttc     240

cagggagcct gcaaactgac cacacaccgc tgttatccct tcctcacgcc agtgggtacc     300

agcctgcctt gatgttttca ccaacccagc ctggaagacc acatacagga aacgtagcca     360

ttccccaggt gacctctgtc gaatcaaagc ccctaccgcc tcttgccttc aaacacacag     420

ttggacacat aatactttct gaacataaag gtgtcaaatt taattgctca atcagtgtac     480

ctaatatata ccaggacacc acaatttctt ggtggaaaga tgggaaggaa ttgcttgggg     540

cacatcatgc aattacacag ttttatccag atgatgaagt tacagcaata atcgcttcct     600

tcagcataac cagtgtgcag cgttcagaca atgggtcgta tatctgtaag atgaaaataa     660

acaatgaaga gatcgtgtct gatcccatct acatcgaagt acaaggactt cctcacttta     720

ctaagcagcc tgagagcatg aatgtcacca gaaacacagc cttcaacctc acctgtcagg     780

ctgtgggccc gcctgagccc gtcaacattt tctgggttca aaacagtagc cgtgttaacg     840

aacagcctga aaaatccccc tccgtgctaa ctgttccagg cctgacggag atggcggtct     900

tcagttgtga ggcccacaat gacaaagggc tgaccgtgtc caagggagtg cagatcaaca     960

tcaaagcaat tccctcccca ccaactgaag tcagcatccg taacagcact gcacacagca    1020

ttctgatctc ctgggttcct ggttttgatg gatactcccc gttcaggaat tgcagcattc    1080

aggtcaagga agctgatccg ctgagtaatg gctcagtcat gatttttaac acctctgcct    1140

taccacatct gtaccaaatc aagcagctgc aagccctggc taattacagc attggtgttt 1200

cctgcatgaa tgaaataggc tggtctgcag tgagcccttg gattctagcc agcacgactg 1260

aaggagcccc atcagtagca cctttaaatg tcactgtgtt tctgaatgaa tctagtgata 1320

atgtggacat cagatggatg aagcctccga ctaagcagca ggatggagaa ctggtgggct 1380

accggatatc ccacgtgtgg cagagtgcag ggatttccaa agagctcttg gaggaagttg 1440

gccagaatgg cagccgagct cggatctctg ttcaagtcca caatgctacg tgcacagtga 1500

ggattgcagc cgtcaccaga gggggagttg ggcccttcag tgatccagtg aaaatattta 1560

tccctgcaca cggttgggta gattatgccc cctcttcaac tccggcgcct ggcaacgcag 1620

atcctgtgct catcatcttt ggctgctttt gtggatttat tttgattggg ttgattttat 1680

acatctcctt ggccatcaga aaaagagtcc aggagacaaa gtttgggaat gcattcacag 1740

aggaggattc tgaattagtg gtgaattata tagcaaagaa atccttctgt cggcgagcca 1800

ttgaacttac cttacatagc ttgggagtca gtgaggaact acaaaataaa ctagaagatg 1860

ttgtgattga caggaatctt ctaattcttg gaaaaattct gggtgaagga gagtttgggt 1920

ctgtaatgga aggaaatctt aagcaggaag atgggacctc tctgaaagtg gcagtgaaga 1980

ccatgaagtt ggacaactct tcacagcggg agatcgagga gtttctcagt gaggcagcgt 2040

gcatgaaaga cttcagccac ccaaatgtca ttcgacttct aggtgtgtgt atagaaatga 2100

gctctcaagg catcccaaag cccatggtaa ttttaccctt catgaaatac ggggacctgc 2160

atacttactt actttattcc cgattggaga caggaccaaa gcatattcct ctgcagacac 2220

tattgaagtt catggtggat attgccctgg gaatggagta tctgagcaac aggaattttc 2280

ttcatcgaga tttagctgct cgaaactgca tgttgcgaga tgacatgact gtctgtgttg 2340

cggacttcgg cctctctaag aagatttaca gtggcgatta ttaccgccaa ggccgcattg 2400

ctaagatgcc tgttaaatgg atcgccatag aaagtcttgc agaccgagtc tacacaagta 2460

aaagtgatgt gtgggcattt ggcgtgacca tgtgggaaat agctacgcgg ggaatgactc 2520

cctatcctgg ggtccagaac catgagatgt atgactatct tctccatggc cacaggttga 2580

agcagcccga agactgcctg gatgaactgt atgaaataat gtactcttgc tggagaaccg 2640

atcccttaga ccgccccacc ttttcagtat tgaggctgca gctagaaaaa ctcttagaaa 2700

gtttgcctga cgttcggaac caagcagacg ttatttacgt caatacacag ttgctggaga 2760

gctctgaggg cctggcccag ggctccaccc ttgctccact ggacttgaac atcgaccctg 2820

actctataat tgcctcctgc actccccgcg ctgccatcag tgtggtcaca gcagaagttc 2880

atgacagcaa acctcatgaa ggacggtaca tcctgaatgg gggcagtgag gaatgggaag 2940

atctgacttc tgccccctct gctgcagtca cagctgaaaa gaacagtgtt ttaccggggg 3000

agagacttgt taggaatggg gtctcctggt cccattcgag catgctgccc ttgggaagct 3060

cattgcccga tgaacttttg tttgctgacg actcctcaga aggctcagaa gtcctgatgt 3120

gaggagaggt gcggggagac attccaaaaa tcaagccaat tcttctgctg taggagaatc 3180

caattgtacc tgatgttttt ggtatttgtc ttccttacca agtgaactcc atggccccaa 3240

agcaccagat gaatgttgtt aagtaagctg tcattaaaaa tacataatat atatttattt 3300

aaagagaaaa aatatgtgta tatcatggaa aaagacaagg atattttaat aaaacattac 3360

ttatttcatt tcacttatct tgcatatctt aaaattaagc ttcagctgct ccttgatatt 3420

aacatttgta cagagttgaa gttgtttttt caagttcttt tctttttcat gactattaaa 3480

tgtaaaaata tttgtaaaat gaaatgccat atttgacttg gcttctggtc ttgatgtatt 3540

tgataagaat gattcattca atgtttaaag ttgtataact gattaatttt ctgatatggc 3600

ttcctaataa aatatgaata aggaagaaaa aa 3632

<210> 2
<211> 5198
<212> DNA
<213> Homo sapiens

<220>
<221> misc_feature
<223> cullin 4B (CUL4B)

<400> 2
   agactcgctg agtcgctcct gcagaaaggg ggggagagag atcgaaaagc aggggagggg 60
   gacggcacgg ccgtttacct gtctgcctcc tcattcgctc tcccccctcg ttctgctcac 120
   tcctggtgtc agcctatccg ccttcccaaa ccctcccatt cccccggtgt agcccccccc 180
   ttcactttcc ttctcgtcct ctgtgtttct cctctcttct ttcttccctt ccccctctag 240
   cattgctacc ttctctccta cacgcacgca ggcatataaa cgtaggtttt tgatgctcct 300
   ctgcctgttg accccgctat tttcatgttt ccaacaggtt tttcttcccc cagtccctca 360
   gctgctgctg ctgctcagga ggtcagatct gccactgatg gtaataccag caccactccg 420
   cccacctctg ccaagaagag aaagttaaac agcagcagca gtagcagcag taacagtagt 480
   aacgagagag aagactttga ttccacctct tcctcctctt ccactcctcc tttacaaccc 540
   agggattcgg catccccttc aacctcgtcc ttctgcctgg gggtttcagt ggctgcttcc 600
   agccacgtac cgatacagaa gaagctgcgt tttgaagaca ccctggagtt tgtagggttt 660
   gatgcgaaga tggctgagga atcctcctcc tcctcctcct catcttcacc aactgctgca 720
   acatctcagc agcagcaact taaaaataag agtatattaa tctcttctgt ggcttcggtg 780
   catcatgcaa acggcctagc caaatcttct accaccgtct ctagctttgc taacagcaaa 840
   cctggctctg ctaagaagtt agtgatcaag aactttaaag ataagcctaa attaccagaa 900
   aactacacag atgaaacctg gcaaaaactg aaagaagcag tggaagctat tcagaatagt 960
   acttcaatta agtacaattt agaagaactc taccaggctg tagaaaatct ctgttcttac 1020
   aagatttctg caaacttgta caaacagctg agacagatct gcgaagatca catcaaagca 1080
   cagattcatc aattcagaga ggattcattg gatagcgttc tttttttaaa gaagattgat 1140
   agatgctggc aaaaccattg cagacaaatg atcatgatca ggagcatttt tttgtttctg 1200
   gatagaactt acgttcttca gaattcaatg ctaccctcca tttgggacat gggactggag 1260
   ttatttaggg ctcatattat aagtgatcag aaagtgcaga ataagacaat tgatggcatt 1320
   cttctcttga ttgagaggga aaggaatggt gaagcaattg atagaagttt acttcgaagc 1380
   cttttaagca tgctgtctga tttgcaaatt tatcaagatt cttttgaaca acgatttttg 1440
   gaagaaacta accggctcta tgcagctgaa ggccaaaaat taatgcaaga aagagaggtt 1500
   cctgaatatc tacatcatgt taacaaacgt ctagaagaag aagcagacag acttattact 1560
   tacttagatc agaccaccca gaagtcatta attgctactg tagaaaaaca acttctaggt 1620
   gaacacttaa cagcaattct tcagaaaggt ttaaataacc tccttgatga aaaccgaatt 1680
   caagatttgt ctcttctgta tcagctcttc agtagagttc gaggtggagt tcaggttctt 1740
   ttgcagcagt ggatcgaata tatcaaggca tttggcagca ctattgtaat taatcctgaa 1800
   aaagataaaa ccatggttca agaattgctg gattttaaag ataaggttga ccatataatt 1860
   gatatctgct ttctgaagaa tgagaaattt atcaatgcca tgaaagaagc atttgaaacg 1920
   ttcattaaca aaagaccaaa taaaccagct gaacttatag ctaagtatgt agattcaaaa 1980
   cttcgtgcag gcaacaaaga agctacagat gaagaacttg agaaaatgtt ggataaaatt 2040
   atgatcatat ttagatttat ctatggcaag gatgtttttg aggccttcta taagaaagat 2100
   ttagccaagc gcctgttagt cggaaagagt gcatctgtag atgctgaaaa atcaatgctg 2160
   tccaaactta aacatgaatg cggagctgct ttcaccagca aacttgaagg aatgtttaaa 2220
   gacatggaac tttctaaaga catcatgatt cagttcaaac agtatatgca gaatcagaat 2280
   gttccgggaa atattgagtt aactgtgaat atcctgacaa tgggctattg gccgacatat 2340
   gtgcctatgg aagttcattt accaccagag atggtaaaac ttcaggagat tttcaagaca 2400
   ttttacctag gcaaacatag tggcaggaaa cttcagtggc agtcaaccct aggacactgt 2460
   gtgttaaaag cagaatttaa agagggtaaa aaggaactcc aggtctctct ttttcaaaca 2520
   ctggtgctgc taatgtttaa tgagggagag gagttcagtt tagaagagat caagcaggca 2580
   actggaatag aggatggaga gttaaggaga acactgcagt cattagcctg tggcaaagct 2640
   agagttctgg cgaaaaatcc aaagggcaaa gacattgaag atggtgacaa gttcatttgt 2700
   aatgatgatt tcaaacataa acttttcagg ataaagatca atcaaatcca gatgaaagaa 2760
   acggttgaag aacaagcaag cactacagaa agagtatttc aagacagaca gtatcaaatt 2820
   gatgctgcaa ttgttcgaat tatgaagatg agaaagacac ttagccacaa tctccttgtt 2880
   tcagaagtgt acaaccagtt gaaatttcca gtaaagcctg ctgatcttaa gaagagaata 2940
   gaatctttaa ttgaccggga ctacatggaa agagataaag aaaatccaaa ccagtacaac 3000
   tatattgcat agaatgttgg ccttgcagca tttggtgtca tatgcagtag ccagtggata 3060
   aactaacctg ttgattcaat attatttgac tcctttttta ctaccgatga ccaaatgaag 3120
   cagtgtaatt aatggaaata gttgagtgga ctttttctca gtggttaaca tgcccatttt 3180
   aaagagtaat acttaccttt aagaagaatg ttgttgaact ctttgcatgt tatttagtat 3240
   gatgtgcaga aaacacttaa gaacgtacct ggtcttcatg aattcctctt tggaactggg 3300
   aaagagatcc ctgtggctat taaaaagggg ggagggttct tacacaccat taattatgaa 3360
   gcaaaaggtt tatttgctta aaatgtcatt taaagatact taaactgcat gcaaacttta 3420
   tttactgtac agagttctgg atgtatttat caaatagaaa aaccaccctt gacttggttg 3480
   ttcacccgtt ttgtgatttc ccttgaaaag agaaatgaat tgtcatagct attgatattt 3540
   tactaggtga tgttctgtta cactgaaagg gatgtttttt aaaaaaaatt atttggaaac 3600
   aagttttcaa gtagggggac agttgcttac ataatatctt gtatattcac atcctaaaaa 3660
   ttttcattta tgagtatggg atgtgtgtat atggcttagc cccaagttaa ctgtgctgat 3720
   taacaagtac ttataaaaat agctaaagta gaaaattaat gatcagttgt actagaggaa 3780
   catcatagaa gagaaaaatc caactcttta tattaaaaat gaatacaata agaaaattgc 3840
   aaaagagacc aaaatgaacg tgttttgtaa attgttacag gagcaaaaac tttgcaacta 3900
   gttttcaaca tactttagac tttaaatgtt gtttggacag ttttgtcttc aacttggacc 3960
   aattgtagat tgttacaggc ttcctaggat ttagcattcc tattttctct accaaagttt 4020
   tttttagaag catcccaact cttctctttg ttgataaata agcctttcct gtcatcttca 4080
   ttttaacttg ctaaaaccct agatacaaag gtcctccctc atttttccca cagagcccct 4140
   gtttgggaat ttgagattct gtgtcatgct ggttttcctt tcagaattgc ttgaaatcga 4200
   gttctcttag ccactggaca cctagttttc aggagaatgt cccaagattg tttatatata 4260
   caaaatggca aaagtactgt ttttcgaatt gataattcaa gcaaaggctt ctctttactg 4320
   tcactgattt ttttttgttt ttattttgtt tccttgtttc tatttccttg aagctttgaa 4380
   gggaggagaa gcagaaaatt ttgtttatgt ttgttaaaaa tcagaactac ttaatgcttt 4440
   ttgccacagc atatttcctt gcctgttgga gccgttagga agattaccta tgtcctaatc 4500
   ttcaacctga gaagattttt gactcttgtg tctactgttg attgtttgct tgagttctca 4560
   aagacctgtg gccaggtatt tttaaaagtc gtctatttct ttctagttgc atcaacttgc 4620
   acactggtgt ctaactatat gtggtattcg gaacttgtaa ttcttattga gcagccattg 4680
   cctatatttt ctcagctcct cactgatttc tttatatgag ctcagctgac aagcaaatgc 4740
   agctattttg tttaccctct cttcataccg gaccatcaac aacagtgttc taactcatcc 4800
   ctgatggtct gtgagatgta gactattaca caagattcaa gttactcgta agcaactttt 4860
   agatagagtt ggtcctgtta ggaggagact cttgatgtca ccttcagtat cttgaaagcg 4920
   ggtcccctcc ccgaggctct taattctttg aaaacttgat gctgtttcag ctgaaaaatt 4980
   agcaagactg ttaaaaaaaa aaagttgagg ggagctgttt aagaaactga aaagtaattg 5040
   caaactacat tggataattg tgactttcag ttgttgttct gtatcagttg aatttttgtg 5100
   ctcttttccc tgtgtacgtg gtggttctat ttttctccaa taaaactttt atttaaaaaa 5160
   agtttctaga gaagatcttt aaaaaaaaaa aaaaaaaa 5198

<210> 3
<211> 1739
<212> DNA
<213> Homo sapiens

<220>
<221> misc_feature
<223> lectin, galactoside-binding, soluble, 9 (LGALS9)

<400> 3
tccccattga ataacagcca agttgctttg gtttctattt ctttgttaag tcgttccctc 60

tacaaaggac ttcctagtgg gtgtgaaagg cagcggtggc cacagaggcg gcggagagat 120

ggccttcagc ggttcccagg ctccctacct gagtccagct gtcccctttt ctgggactat 180

tcaaggaggt ctccaggacg gacttcagat cactgtcaat gggaccgttc tcagctccag 240

tggaaccagg tttgctgtga actttcagac tggcttcagt ggaaatgaca ttgccttcca 300

cttcaaccct cggtttgaag atggagggta cgtggtgtgc aacacgaggc agaacggaag 360

ctgggggccc gaggagagga agacacacat gcctttccag aaggggatgc cctttgacct 420

ctgcttcctg gtgcagagct cagatttcaa ggtgatggtg aacgggatcc tcttcgtgca 480

gtacttccac cgcgtgccct tccaccgtgt ggacaccatc tccgtcaatg gctctgtgca 540

gctgtcctac atcagcttcc agcctcccgg cgtgtggcct gccaacccgg ctcccattac 600

ccagacagtc atccacacag tgcagagcgc ccctggacag atgttctcta ctcccgccat 660

cccacctatg atgtaccccc accccgccta tccgatgcct ttcatcacca ccattctggg 720

agggctgtac ccatccaagt ccatcctcct gtcaggcact gtcctgccca gtgctcagag 780

gttccacatc aacctgtgct ctgggaacca catcgccttc cacctgaacc cccgttttga 840

tgagaatgct gtggtccgca acacccagat cgacaactcc tgggggtctg aggagcgaag 900

tctgccccga aaaatgccct tcgtccgtgg ccagagcttc tcagtgtgga tcttgtgtga 960

agctcactgc ctcaaggtgg ccgtggatgg tcagcacctg tttgaatact accatcgcct 1020

gaggaacctg cccaccatca acagactgga agtggggggc gacatccagc tgacccatgt 1080

gcagacatag gcggcttcct ggccctgggg ccgggggctg gggtgtgggg cagtctgggt 1140

cctctcatca tccccacttc ccaggcccag cctttccaac cctgcctggg atctgggctt 1200

taatgcagag gccatgtcct tgtctggtcc tgcttctggc tacagccacc ctggaacgga 1260

gaaggcagct gacggggatt gccttcctca gccgcagcag cacctggggc tccagctgct 1320

ggaatcctac catcccagga ggcaggcaca gccagggaga ggggaggagt gggcagtgaa 1380

gatgaagccc catgctcagt cccctcccat cccccacgca gctccacccc agtcccaagc 1440

caccagctgt ctgctcctgg tgggaggtgg cctcctcagc ccctcctctc tgacctttaa 1500

cctcactctc accttgcacc gtgcaccaac ccttcacccc tcctggaaag caggcctgat 1560

ggcttcccac tggcctccac cacctgacca gagtgttctc ttcagaggac tggctccttt 1620

cccagtgtcc ttaaaataaa gaaatgaaaa tgcttgttgg cacattcaaa aaaaaaaaaa 1680

aaaaaaaaa aaaaaaaaa aaaaaaaaa aaaaaaaaa aaaaaaaaa aaaaaaaa 1739

<210> 4
<211> 3482
<212> DNA
<213> Homo sapiens

<220>
<221> misc_feature
<223> nuclear transcription factor Y, beta (NFYB)

<400> 4
ggagggaggg gaggccgagt cctggaagcg gagctccgcg ctgggactgg ttccttcgca 60

gccattttct gtccaaccaa acagccgatt ggagacggga gccaaccagg gctgcattgg 120

aggttgaaat cacaaagatt agacaccttt ttagataggt gttcttcagc accactgaca 180

acacggttct gacagtattt catgacaatg gatggtgaca gttctacaac agatgcttct 240

caactaggaa tctctgcaga ctatattgga ggaagtcatt atgttataca gcctcatgat 300

gatactgagg acagcatgaa tgatcatgaa gacacaaatg gttcaaaaga aagtttcaga 360

gaacaagata tatatcttcc aatagcaaac gtggctagga taatgaaaaa tgccatacct 420

caaacgggaa agattgcaaa agatgccaaa gaatgtgttc aagaatgtgt aagtgagttc 480

atcagtttta taacatctga agcaagtgaa aggtgccatc aagagaaacg gaaaacaatc 540

aatggagaag atattctctt tgctatgtct actttaggct ttgacagtta tgtggaacct 600

ctgaaattat accttcagaa attcagagag gctatgaaag gagaaaaggg aattggtgga 660

gcagtcacag ctacagatgg actaagtgaa gagcttacag aggaggcatt tactaaccag 720

ttaccagctg gcttaataac cacagacggt caacaacaaa atgttatggt ttacacaaca 780

tcatatcaac agatttctgg tgttcagcaa attcagtttt catgatctga agaaatgatg 840

gaatggggag tgtagagaaa tgagagtctg tatgattctg gaacagagac atcagaagga 900

aagactggtg aaaagatgta tctttgtata ttaatagctg taatgtagct tcctgatgct 960

tgactaattg aggtgttaat tctgacttga gaatcttttt catgaatgat tttaaagaaa 1020

aatttggatt ttaaaggtat taaaatattt ttgttttgta cgagagtttg ttgctctgta 1080

tgactcctgt atgcattgta tattgcaatt tattactgtc agagatttgt agacagtttc 1140

ttattttcat attgaatcat gttacttttg taattcaagt aagcggctgg gttaattcat 1200

gatgtttgcc cttttaataa aatataaggg tagagttcat tttgaatgca agttgccttt 1260

attataaatt tgagtttgtc ttggttatac cttgcatgat aacctagcta gatttctagc 1320

atttgctgta tttattaaaa ttattatttt tttggtaaaa cattaatagt ttaagcagca 1380

tcattttttt aaaaaatgta attgaataag tgtgaatgca gaagcaaata ttgtctgccc 1440

tgttaaactt ggtgcccatt aacagtgttt acactgttca tcgtgcctgt taatgtagtt 1500

ttagttactg gagctttttt aagactagat ttggttttga gttacatttt taagaatgtg 1560

ggaatatatt taagtttaat gtagtcctag tgctcttgaa atggtgcccc tttcatttgg 1620

tacatgattt tttttcaaat catatcttca agtactatag tattctctta cagaagagga 1680

gttttatagt ctgatggtaa atgtcttcat tttacctttt taattgaaat gtcaagtttc 1740

ctgttacact atggaaacca agaaacatca gacatcattg cgtgtacaga ccttttgcat 1800

gggtgagtgg atgaaatgga gaacagagtg agtgctgtga acggtgtgaa atagaagcca 1860

acttctagta tgctgtcttc atctctgcaa taaactaaac gtaaataatg taactttgta 1920

taatttagta gtatgtactt ggattgtttc tttttgaaaa gtgaggattt gtattttctc 1980

atctattctt tttggtgtta attaacttga gggatttctt ttttaagttt ttaatatcaa 2040

aaattgctat tgtatagttt gcctaaaagg aagttggagt gaatatcatc tttgaaggac 2100

actcaaattt ataaggtaga agcaatttcc cagatatagg ctgaacagaa acatttactt 2160

tgccagaagg agcctttagg agtatatgtg ggctaatcag aggttaataa tgtcctcatc 2220

tcctactctc catttaaaaa acaaacttca ttgtagtaaa ttcaaatagg aaaaaatggg 2280

ggagtagtta agcaaaatca acagtcatcc tactatgttg aaagaaccaa tgttaatatt 2340

ttgataaata tcatttgtcc ttttactttg ctaaatatgt atatttttat aaaaatgggc 2400

tcatattgtg cataccattt tgtaacctgc ttttttcact taacaatata ttgggaacat 2460

cttttccagg tcatgcatag tcttctgcat catttttatt ggtttattat tctgctgtat 2520

gactcaatca taattcattt aatcttctat tggatatttt gactatttac atcttttcac 2580

agttataaac agcacttcac tgaatcccat gtacatccat gactgttttc ttaaattctt 2640

tgaattgctg ggttaccaaa tatgcaaaat tttaaggctt ttgatacttc gttatgagtg 2700

tatttttgag atattttcca cttgatccaa gcagtgtgtc actatataac tgataaaagc 2760

acttggtctt tgattatcag atactgtctc taaaattatt ttaaagatta atatttgttc 2820

aagtagagaa tgaagataaa aaaggaattt gaggccaggt atggggtgtc tcatgcctat 2880

aattccagca ctttgggagg ccaaggcagg cagatcactt tgaggtcagg agtttgagac 2940

cagcctggcc aacatggaga accccccgtc tctacaaaaa atacaaaatt agcagggtgt 3000

ggtggtgcac acctgtagtc ccttactccc tggtggctga ggcattatcc cttgaacctg 3060

ggaggcagag cctaaagtga gccgagatcg cgccactgca ctccagcctg ggtgacacag 3120

caagactctt gtctcaaaca aacaggaatt tgagtaaatt ttaaattagt atatactaca 3180

acaaatttcc aaacttcata atttgctagt agtgctaact aaaattttac accaggtaaa 3240

atcaggtcac cttttttatg tattgacttc attgttggac ttcatactct gatgggaaag 3300

aaaatggaca ttctgaatgt gtcaggtgca taatatccta atttatgctt gtcagagctg 3360

gggtctctgt cttccttggc ccatgttgca tttaactgaa tgtcatgttt tctcctctgg 3420

aacttcttat gtatgcaagc tcccatgtta acctatcata aaaatgtttt ttataatttt 3480

gt 3482

<210> 5
<211> 7221
<212> DNA
<213> Homo sapiens

<220>
<221> misc_feature
<223> phospholipase C, beta 1 (phosphoinositide-specific) (PLCB1)

<400> 5
cactcgctct ctccctctgt gtatagagga tgtgctgaat ggtgcgcttt gaggcggcgg 60

cggcggagga gcagaatccg ccgcgactgg cagcctcggc tgaccggctc ggcttctctt 120

cgccttccga ggctcctcat ccaccgcggg ctccagacct cgcgtcccgc ccggggcatg 180

gccgggcgct gcgcccccgc gcgctctgcc tgctgagcgg cgccggaggg aggtgcggag 240

gccgggaggc cggggaggcc ggcggggagc agagtcgagc gcctccggag cagagaaagg 300

agcccgcgcc ccgcgccccg cgccccgcgc acggtcccca gtccctgccg cgctcgcccg 360

ggccgcccgg agcccagatg agcccagatg gccggggctc aacccggagt gcacgccttg 420

caactcaagc ccgtgtgcgt gtccgacagc ctcaagaagg gcaccaaatt cgtcaagtgg 480

gatgatgact caactattgt tactccaatt attttgagga ctgaccctca gggatttttc 540

ttttactgga cagatcaaaa caaggagaca gagctactgg atctcagcct tgtcaaagat 600

gccagatgtg ggagacacgc caaagctccc aaggacccca aattacgtga acttttggat 660

gtggggaaca tcgggcgcct ggagcagcgc atgatcacag tggtgtatgg gcctgacctc 720

gtgaacatct cccatttgaa tctcgtggct ttccaagaag aagtggccaa ggaatggaca 780

aatgaggttt tcagtttggc aacaaacctg ctggcccaaa acatgtccag ggatgcattt 840

ctggaaaaag cctatactaa acttaagctg caagtcactc cagaagggcg tattcctctc 900

aaaaacatat atcgcttgtt ttcagcagat cggaagcgag ttgaaactgc tttagaggct 960

tgtagtcttc catcttcaag gaatgattca atacctcaag aagatttcac tccagaagtg 1020

tacagagttt tcctcaacaa cctttgccct cgacctgaaa ttgataacat cttttcagaa 1080

tttggtgcaa aaagcaaacc atatcttacc gttgatcaga tgatggattt tatcaacctt 1140

aagcagcgag atcctcggct taatgaaata ctttatccac ctctaaaaca agagcaagtc 1200

caagtattga ttgagaagta tgaacccaac aacagcctcg ccagaaaagg acaaatatca 1260

gtggatgggt tcatgcgcta tctgagtgga gaagaaaacg gagtcgtttc acctgagaaa 1320

ctggatttga atgaagacat gtctcagccc ctttctcact atttcattaa ttcctcgcac 1380

aacacctacc tcacagctgg ccaactggct ggaaactcct ctgttgagat gtatcgccaa 1440

gtgctcctgt ctggttgtcg ctgtgtggag ctggactgct ggaagggacg gactgcagaa 1500

gaggaacctg tcatcaccca tggcttcacc atgacaactg aaatatcttt caaggaagtg 1560

atagaagcaa ttgcggagtg tgcatttaag acttcacctt ttccaattct cctttcgttt 1620

gagaaccatg tggattcccc aaagcagcaa gccaagatgg cggagtactg ccgactgatc 1680

tttggggatg cccttctcat ggagcccctg gaaaaatatc cactggaatc tggagttcct 1740

cttccaagcc ctatggattt aatgtataaa attttggtga aaaataagaa gaaatcacac 1800

aagtcatcag aaggaagcgg caaaaagaag ctctcagaac aagcctccaa cacctacagt 1860

gactcctcca gcatgttcga gccctcatcc ccaggagccg gagaagctga tacggaaagt 1920

gacgacgacg atgatgatga tgactgtaaa aaatcttcaa tggatgaggg gactgctgga 1980

agtgaggcta tggccacaga agaaatgtct aatctggtga actatattca gccagtcaag 2040

tttgagtcat ttgaaatttc aaaaaaaaga aataaaagtt ttgaaatgtc ttccttcgtg 2100

gaaaccaaag gacttgaaca actcaccaag tctccagtgg aatttgtaga atataacaaa 2160

atgcagctta gcaggatata tccaaaagga acacgtgtgg attcatccaa ctatatgcct 2220

cagctcttct ggaatgcagg ttgtcagatg gtggcactta atttccagac aatggacctg 2280

gctatgcaaa taaatatggg gatgtatgaa tacaacggga agagtggcta cagattgaag 2340

ccagagttca tgaggaggcc tgacaagcat tttgatccat ttactgaagg catcgtagat 2400

gggatagtgg caaacacttt gtctgttaag attatttcag gtcagtttct ttctgataag 2460

aaagttggga cttacgtgga agtagatatg tttggtttgc ctgtggatac aaggaggaag 2520

gcatttaaga ccaaaacatc ccaaggaaat gctgtgaatc ctgtctggga agaagaacct 2580

attgtgttca aaaaggtggt tcttcctact ctggcctgtt tgagaatagc agtttatgaa 2640

gaaggaggta aattcattgg ccaccgtatc ttgccagtgc aagccattcg gccaggctat 2700

cactatatct gtctaaggaa tgaaaggaac cagcctctga cgctgcctgc tgtctttgtc 2760

tacatagaag tgaaagacta tgtgccagac acatatgcag atgtcatcga agctttatca 2820

aacccaatcc gatatgtgaa cctgatggaa cagagagcta agcaattggc tgctttgaca 2880

ctggaagatg aagaagaagt aaagaaagag gctgatcctg gagaaacacc atcagaggct 2940

ccaagtgaag cgagaacgac tccagcagaa aatggggtga atcacactac aaccctgaca 3000

cccaagccac cctcccaggc tctccacagc cagccagctc caggttctgt aaaggcacct 3060

gccaaaacag aagatcttat tcagagtgtc ttaacagaag tggaagcaca gaccatcgaa 3120

gaactaaagc aacagaaatc gtttgtgaaa cttcaaaaga aacactacaa agaaatgaaa 3180

gacctggtta agagacacca caagaaaacc actgacctta tcaaagaaca cactaccaag 3240

tataatgaaa ttcagaatga ctacttgaga aggagagccg ctttggaaaa gtccgccaaa 3300

aaggacagta agaaaaaatc ggaacccagc agccctgatc atggttcatc aacgattgag 3360

caagacctcg ctgctctgga tgctgaaatg acccaaaagt taatagactt gaaggacaaa 3420

caacagcagc agctgcttaa tcttcggcaa gaacagtatt atagtgaaaa ataccagaag 3480

cgagaacata ttaaactgct tattcaaaag ttgacggatg tcgcagaaga gtgtcagaac 3540

aatcagttaa agaagctcaa agaaatctgt gagaaagaaa agaaagaatt aaagaagaaa 3600

atggataaaa agaggcagga gaagataaca gaagctaaat ccaaagacaa aagtcagatg 3660

gaagaggaga agacagagat gatccggtca tatatccagg aagtggtgca gtatatcaag 3720

aggctagaag aagcgcaaag taaacggcaa gaaaaactcg tagagaaaca caaggaaata 3780

cgtcagcaga tcctggatga aaagcccaag ggggaaggtt cctcctcatt cttgtcggaa 3840

acttgccatg aggatccctc tgtttccccc aactttactc cccccaaccc tcaagctctc 3900

aagtggtgac caccgtcctt ccggccagct gcaggtggag ctggagcaag aataccaaga 3960

caaattcaaa agactgcccc tcgagatttt ggaattcgtg caggaagcca tgaaaggaaa 4020

gatcagtgaa gacagcaatc acggttctgc ccctctctcc ctgtcctcag accctggaaa 4080

agtgaaccac aagactccct ccagtgagga gctgggagga gacatcccag gaaaagaatt 4140

tgatactcct ctgtgaatgc tcctgccagg ccttcagaaa ttgcatggcc actccagcgt 4200

catcggactc tctcttatta caaagatcac tgcccaggac catcttcccg agaagcatcc 4260

cttagcctaa aatccacacc aaagggagag ttccagagga atccatgaag aattcccatg 4320

cccaggctcc atgtgtcatg tggaaacctc cacaggtctg ctagtgaaga atgcatgtat 4380

gtgagatttt tgttttcttt ccaatagcaa attcaaagca agcaacttgc aggctccatg 4440

gaacttttaa tgaaggacag tgtcttcttt gaagaaaatc aagctcgtgt ttttattcga 4500

agctctggtg taaaatattt caaagtcata gaaatagttt gagaaatgca tagcattatt 4560

taacactatt gaacagccga ctttgagcat tgtttcttct aactgcccct caactaccat 4620

tatcttcaag tcaacatgca tattacattt tcatcctttg ctttgcaagc actggtggct 4680

tgcagtttgc taatttattt atcatagagt catcaatgta tttgttgctg acatggtttt 4740

attagatact gtagtgattc aaataagttt tctatttgaa aaaaaaaatc acttgattgt 4800

atccttgccc agtgaagcca tcctaagact tagcaatatg gattgtacat ttggctgcat 4860

gagcaagtcg gccgcacact tccagacagt gtgctgtttg aattgactat ttgcactcaa 4920

agtctgggta ttcattggtt attggcctga aatgatcaaa taactacaaa tgatctgttg 4980

aataaaaata gttgagctga tatatgttaa gcagatattc aatcagaatg aacaggttcc 5040

ggtggttatt ttgccgtttg acatttttta tggttcattt atttttaata tagagaggaa 5100

gattgaatat ttatctagag aatacaaaga cccacatgta aatgataggt attatctcca 5160

tgtatatatg tacccactta gtcatgtaag tgcatataca catacacaca catgagtgta 5220

gacatgtgtt tattaattga caatgaccca aatctcttcc acaagactta aaaccaaatt 5280

cagggacaaa tggatagaga agaaaagggt caaacatcga gattacatgg atgttaaatt 5340

atatggagac gctaagaaat aattgatgga gccattgatg caaaccgaag tagatttaga 5400

acttatatga atttgattta tattttgcaa gatcaaaaat tagatgttaa gtatcagatt 5460

ttaagcttgt tttaatggtc aaaaaattag gacagaaata atatggacat ttattagtat 5520

cttccataat ttttaagtct gacacatttc tattttattc taacataaaa aaacttccat 5580

tatatattta ctaagtatat ttaattcact taactctgtc tttataagtt cctattttag 5640

atggaataag agaaacaaat tatatcaagg taaaactgat caaaagcata attgaaagtt 5700

ctgaaaaaag aaaaataata atatgtagaa aaatgtaact tagagagtaa cacatgaaca 5760

ttgaagttaa aacccagaag ccagatgctc acagttttat tttactttaa aataaacctg 5820

tctgactgta gctttgtgaa atatcttaaa acgcaaaaac caattgtgtc ctgaaaattg 5880

tttcaagaat ttaatatttt tatgaaaatt attttattta actttaagca ataactagag 5940

attacaatta aattttaatc aaaatgaagg cttagttcaa acataaggaa acagtgtttg 6000

attaaaaaaa acacatctag taagacgtaa ggggaaaatc acatcctctt tggagatgat 6060

tatattttga tctgaagggt ttggggtgtt cattagacac ttaataaaac ttaacttcca 6120

atgaaaaaga aatcttttgt aaatcattct acttttgcac tttgaaagaa aggcgttaat 6180

cataaagaag caagaatggt caaaatcgaa tgctgcattt ttataaacaa aattacagac 6240

tgtctgaaat tgaagaagaa tgaactaata atagcattca taaccaaaca caatgatgat 6300

tattgcagaa cattgtatca cattttagtc cagagataga ataaagttga ataaccttga 6360

cttacacaaa actgttttgg tagttggatt tcattatctt agtgaattta gtcattttac 6420

aatatgtttg tatttggcca tttactgtaa tcacattttt atatctgtac aatgacactt 6480

tttgcagttg tggggtagtg tgtaacactg tccatcttgc atcattgaaa ctactacaat 6540

gatactatca tttaataata ttaatattac ttgaaataga ctaagataaa gaaaaggggt 6600

ctgtatgatg tgcagttttg tgcctttatg tatttgcctt gttctttgtc gaatgtgtga 6660

aattccgtac tgtggttttt cctataatag aaagtagagc tgtgtattaa attagactgt 6720

gtctctctga tacctttaca ctactgagaa tagcatggtt ttggccatgt aaaccaattt 6780

tcaaagttct aatgacatag ccatgtgttt ttggtttttt atatttcatt ttaaaatttg 6840

agtatcacca tacattaatt aatactcctg tagtagataa gctgtcatta agtaattccc 6900

aaaaaaaggg ccatttgctt gcattacttt gaatttaatg ttgcgcttgt gcactgtgtt 6960

aatattgttt gtgatggatt ggacgttgtg actcttgcct tttaagaaga aaaaaaagat 7020

aggacaaagt atttgaagct cttaaaatgt acatattttg gttcttctat ctcaaattat 7080

ttaaaatgca taattcacat ttttgtaata attctatgca attttgtggc atgatgtttc 7140

ttccacttgt aattttatgt gctttcatca caaatccaaa ggaaagaata aaaatttctt 7200

aacacaaaaa aaaaaaaaaa a 7221

<210> 6
<211> 3687
<212> DNA
<213> Homo sapiens

<220>
<221> misc_feature
<223> solute carrier family 2 (facilitated glucose transporter), member
      1 (SLC2A1)

<400> 6
tccaccattt tgctagagaa ggccgcggag gctcagagag gtgcgcacac ttgccctgag 60

tcacacagcg aatgccctcc gcggtcccaa cgcagagaga acgagccgat cggcagcctg 120

agcgaggcag tggttagggg gggccccggc cccggccact cccctcaccc cctccccgca 180

gagcgccgcc caggacaggc tgggccccag gccccgcccc gaggtcctgc ccacacaccc 240

ctgacacacc ggcgtcgcca gccaatggcc ggggtcctat aaacgctacg gtccgcgcgc 300

tctctggcaa gaggcaagag gtagcaacag cgagcgtgcc ggtcgctagt cgcgggtccc 360

cgagtgagca cgccagggag caggagacca aacgacgggg gtcggagtca gagtcgcagt 420

gggagtcccc ggaccggagc acgagcctga gcgggagagc gccgctcgca cgcccgtcgc 480

cacccgcgta cccggcgcag ccagagccac cagcgcagcg ctgccatgga gcccagcagc 540

aagaagctga cgggtcgcct catgctggcc gtgggaggag cagtgcttgg ctccctgcag 600

tttggctaca acactggagt catcaatgcc ccccagaagg tgatcgagga gttctacaac 660

cagacatggg tccaccgcta tggggagagc atcctgccca ccacgctcac cacgctctgg 720

tccctctcag tggccatctt ttctgttggg ggcatgattg gctccttctc tgtgggcctt 780

ttcgttaacc gctttggccg gcggaattca atgctgatga tgaacctgct ggccttcgtg 840

tccgccgtgc tcatgggctt ctcgaaactg ggcaagtcct ttgagatgct gatcctgggc 900

cgcttcatca tcggtgtgta ctgcggcctg accacaggct tcgtgcccat gtatgtgggt 960

gaagtgtcac ccacagccct tcgtggggcc ctgggcaccc tgcaccagct gggcatcgtc 1020

gtcggcatcc tcatcgccca ggtgttcggc ctggactcca tcatgggcaa caaggacctg 1080

tggcccctgc tgctgagcat catcttcatc ccggccctgc tgcagtgcat cgtgctgccc 1140

ttctgccccg agagtccccg cttcctgctc atcaaccgca acgaggagaa ccgggccaag 1200

agtgtgctaa agaagctgcg cgggacagct gacgtgaccc atgacctgca ggagatgaag 1260

gaagagagtc ggcagatgat gcgggagaag aaggtcacca tcctggagct gttccgctcc 1320

cccgcctacc gccagcccat cctcatcgct gtggtgctgc agctgtccca gcagctgtct 1380

ggcatcaacg ctgtcttcta ttactccacg agcatcttcg agaaggcggg ggtgcagcag 1440

cctgtgtatg ccaccattgg ctccggtatc gtcaacacgg ccttcactgt cgtgtcgctg 1500

tttgtggtgg agcgagcagg ccggcggacc ctgcacctca taggcctcgc tggcatggcg 1560

ggttgtgcca tactcatgac catcgcgcta gcactgctgg agcagctacc ctggatgtcc 1620

tatctgagca tcgtggccat ctttggcttt gtggccttct ttgaagtggg tcctggcccc 1680

atcccatggt tcatcgtggc tgaactcttc agccagggtc cacgtccagc tgccattgcc 1740

gttgcaggct tctccaactg gacctcaaat ttcattgtgg gcatgtgctt ccagtatgtg 1800

gagcaactgt gtggtcccta cgtcttcatc atcttcactg tgctcctggt tctgttcttc 1860

atcttcacct acttcaaagt tcctgagact aaaggccgga ccttcgatga gatcgcttcc 1920

ggcttccggc aggggggagc cagccaaagt gacaagacac ccgaggagct gttccatccc 1980

ctgggggctg attcccaagt gtgagtcgcc ccagatcacc agcccggcct gctcccagca 2040

gccctaagga tctctcagga gcacaggcag ctggatgaga cttccaaacc tgacagatgt 2100

cagccgagcc gggcctgggg ctcctttctc cagccagcaa tgatgtccag aagaatattc 2160

aggacttaac ggctccagga ttttaacaaa agcaagactg ttgctcaaat ctattcagac 2220

aagcaacagg ttttataatt tttttattac tgattttgtt atttttatat cagcctgagt 2280

ctcctgtgcc cacatcccag gcttcaccct gaatggttcc atgcctgagg gtggagacta 2340

agccctgtcg agacacttgc cttcttcacc cagctaatct gtagggctgg acctatgtcc 2400

taaggacaca ctaatcgaac tatgaactac aaagcttcta tcccaggagg tggctatggc 2460

cacccgttct gctggcctgg atctccccac tctaggggtc aggctccatt aggatttgcc 2520

ccttcccatc tcttcctacc caaccactca aattaatctt tctttacctg agaccagttg 2580

ggagcactgg agtgcaggga ggagagggga agggccagtc tgggctgccg ggttctagtc 2640

tcctttgcac tgagggccac actattacca tgagaagagg gcctgtggga gcctgcaaac 2700

tcactgctca agaagacatg gagactcctg ccctgttgtg tatagatgca agatatttat 2760

atatattttt ggttgtcaat attaaataca gacactaagt tatagtatat ctggacaagc 2820

caacttgtaa atacaccacc tcactcctgt tacttaccta aacagatata aatggctggt 2880

ttttagaaac atggttttga aatgcttgtg gattgagggt aggaggtttg gatgggagtg 2940

agacagaagt aagtggggtt gcaaccactg caacggctta gacttcgact caggatccag 3000

tcccttacac gtacctctca tcagtgtcct cttgctcaaa aatctgtttg atccctgtta 3060

cccagagaat atatacattc tttatcttga cattcaaggc atttctatca catatttgat 3120

agttggtgtt caaaaaaaca ctagttttgt gccagccgtg atgctcaggc ttgaaatgca 3180

ttattttgaa tgtgaagtaa atactgtacc tttattggac aggctcaaag aggttatgtg 3240

cctgaagtcg cacagtgaat aagctaaaac acctgctttt aacaatggta ccatacaacc 3300

actactccat taactccacc cacctcctgc acccctcccc acacacacaa aatgaaccac 3360

gttctttgta tgggcccaat gagctgtcaa gctgccctgt gttcatttca tttggaattg 3420

ccccctctgg ttcctctgta tactactgct tcatctctaa agacagctca tcctcctcct 3480

tcacccctga atttccagag cacttcatct gctccttcat cacaagtcca gttttctgcc 3540

actagtctga atttcatgag aagatgccga tttggttcct gtgggtcctc agcactattc 3600

agtacagtgc ttgatgcaca gcaggcactc agaaaatact ggaggaaata aaacaccaaa 3660

gatatttgtc aaaaaaaaaa aaaaaaa 3687

<210> 7
<211> 3344
<212> DNA
<213> Homo sapiens

<220>
<221> misc_feature
<223> alpha- and gamma-adaptin binding protein (AAGAB)

<400> 7
attccgagaa cgccctctcc ggcctgtctc cccctcgcgc cggcggccgt ggtgcagccc 60

aaggcggaag cggctgcctg acggaaccgg ctcgcgagcg cagctatggc tgctggcgta 120

ccctgtgcgt tagtcaccag ctgctcctcc gtcttctcag gagaccagct ggtccaacgt 180

gagtagatag gcgcctgggc cgacctccaa ggcccctgag cctcagcggg cgacggaccc 240

agcaccacgg cctctctggg ccttgtcccc gcagcgggcc ctgcgttctt cctccagtca 300

gtggaggcgc ccagtctccc cagatatcct tggaacagaa gatcttattg tggaagtgac 360

ttccaatgat gctgtgagat tttatccctg gaccattgat aataaatact attcagcaga 420

catcaatcta tgtgtggtgc caaacaaatt tcttgttact gcagagattg cagaatctgt 480

ccaagcattt gtggtttact ttgacagcac acaaaaatcg ggccttgata gtgtctcctc 540

atggcttcca ctggcaaaag catggttacc tgaggtgatg atcttggtct gcgatagagt 600

gtctgaagat ggtataaacc gacaaaaagc tcaagaatgg tgcatcaaac atggctttga 660

attggtagaa cttagtccag aggagttgcc tgaggaggat gatgacttcc cagaatctac 720

aggagtaaag cgaattgtcc aagccctgaa tgccaatgtg tggtccaatg tagtgatgaa 780

gaatgatagg aaccaaggct ttagccttct caactcattg actggaacaa accatagcat 840

tgggtcagca gatccctgtc acccagagca accccatttg ccagcagcag atagtactga 900

atccctctct gatcatcggg gtggtgcatc taacacaaca gatgcccagg ttgatagcat 960

tgtggatccc atgttagatc tggatattca agaattagcc agtcttacca ctggaggagg 1020

agatgtggag aattttgaaa gactcttttc aaagttaaag gaaatgaaag acaaggctgc 1080

gacgcttcct catgagcaaa gaaaagtgca tgcagaaaag gtggccaaag cattctggat 1140

ggcaatcggg ggagacagag atgaaattga aggcctttca tctgatgaag agcactgaat 1200

tattcatact agggtttgac caacaaagat gctagctgtc tctgagatac ctctctactc 1260

agcccagtca tattttgcca aaattgccct tatcatgttg gctgcctgac ttgtttatag 1320

ggtcccctta attttagttt ttagtaggag gttaaggaga aatctttttt ttcctcagta 1380

tattgtaaga gagtgaggaa tacagtgata gtaatgagtg aggatttctt aaatatactt 1440

tttttttgtt ctaggaatga gggtaggata aatctcagag gtctgtgtga tttactcaag 1500

ttgaagacaa cctccaggcc attcctggtc aaccttttaa gtagcatttc cagcattcac 1560

acttgatact gcacatcagg agttgtgtca cctttcctgg gtgatttggg ttttctccat 1620

tcaaggagct tgtagctctg agctatgatg cttttattgg gaggaaagga ggcagctgca 1680

gaattgatgt gagctatgtg gggccgaagt ctcagcccgc agctaagtct ctacctaaga 1740

aaatgcctct gggcattctt ttgaagtata gtgtctgagc tcatgctaga aagaatcaaa 1800

aagccagtgt ggatttttag actgtaataa atgaggcaaa ggatttctat tccagtggga 1860

agaaaacctc tctactgagt tgtgggggat atgttgtatg ttagagagaa ccttaaggag 1920

tccttgtatg ggccatggag acagtatgtg ataacatacc gtgattttca tgaagaaatt 1980

cttctgtctt agagttctcc cctgctgctt gagatgccag agctgtgttg ttgcacacct 2040

gcaaaacaag gcacatttcc ccctttctct ttaaagccaa agagagatca ctgccaaagt 2100

gggagcacta aggggtgggt ggggaagtga aatgttaggc gatgaattcc tgagcacctt 2160

gtttttcttc caaggttcgt agctcctctc tgcccttcca agcctgtaac ctcggaggac 2220

tatcttttgt tctttatcct ttgtcttgtt tgagtgggtc agccccagag gaactgataa 2280

gcaaatggca agtttttaaa ggaagagtgg aaagtactgc aaataaaaat ccttatttgt 2340

ttttgtagac tttgtaatgc atatcattag ccctcactgt gatcattact gctgtggctc 2400

tgaactggca catagtacag tggatggaag gtgcccgcac accagctgag aactggttct 2460

ggcctaggtg ggctctagaa ccatttacac agcatgaaag aaacaggttg ggttaggagc 2520

agaaagaaat aaggctcaca cccctccaga cactacctta taagcactgc agaacctgaa 2580

acagatggca gaaggaatgg aatgctacag gggccagcag gagtgaccac agggagggga 2640

cagctcagtg actggagcat tcaggaagag gctttccagg gaacactgga cattgcttag 2700

tgaccttttg ttcctttttt tttttttttc ttttactgtt ctgaaagact ttgagtctgt 2760

ggttcaccac cagcccatca gtgtttcttt gaggtgattg cattagggaa gttggctctg 2820

ggattgcaaa aaaaaaaaaa aggtggaaca tgttttcctt aaaagatgga aggttttaga 2880

aaatatacta ggccatctgg ttagaaaaaa cagaccagac tagaaaaagc tgtgaatttg 2940

attttgtaga ttaaacaaag ccagatgatt aaaatgtgat ttatttataa actgtgctaa 3000

tgtctattat tatgattttg tggtttttta ttagggggta tgagtgaggg aagccctata 3060

tccttcattg gattagacat atatagtgaa tagtggaaga gtctgtcaag ctcactattt 3120

tagaatttac cctgcatatg taatgtcatg tttttaagac ctcagctatg cagggatatt 3180

ggcctcttct agggacatat gggatgaagg tctctagtga aatctactaa aagcccatgt 3240

tagaatagtg atcatgagct aacactacag taatagctgc agtctatcta aagtgaccta 3300

tgttcatgct accacaagtt gttacagaat tgccagctgg aatt 3344

<210> 8
<211> 824
<212> DNA
<213> Homo sapiens

<220>
<221> misc_feature
<223> polymerase (RNA) III (DNA directed) polypeptide K, 12.3 kDa
 (POLR3K)

<400> 8
cggcagtggg agctgccggg agttggagcc tgcggagttc gagaccatgc tgctgttctg 60

ccccggctgc gggaacgggc tgatcgtgga ggagggacaa cgctgccacc gcttctcctg 120

caacacgtgc ccctacgtgc acaacatcac ccgcaaggta acaaatcgga agtacccaaa 180

actgaaagaa gtggatgatg tgcttggtgg agcagctgcc tgggagaatg ttgactctac 240

tgcagagtcg tgtcccaaat gcgaacatcc tcgtgcttac ttcatgcagc ttcagacccg 300

ctctgcagat gagccgatga ccaccttcta caagtgctgc aatgctcagt gtggacaccg 360

ctggagggat tagggccagg atggcccagc tgccctagtg tgtgcttgcc ttgtccctcg 420

gggtagatgc ttagctggca gtatgagttg tgtgtcctga gggtctttgc tagtgtggtg 480

gaaagataaa cctttgaggg tgaagagcca gggggtcagg aaatatggcc tatctgccag 540

gcagggtgga tgaagtcatg aatgtctggg agtttttctg tgtggggagg agacagagac 600

ccataactaa atatgctctg tgtaaagtcc tattctttca tcttccactt tattggcagt 660

tgacattccc ttactcccaa tcaacactct taaatatttg tactgtttgt aaaacttagt 720

acatgtccct aaatatttaa ctgttacttg taaacttgtg taatttatta tttattttaa 780

tcaaaattct gaatatttca tttaaatgaa agttggaata ttgc 824

<210> 9
<211> 1667
<212> DNA
<213> Homo sapiens

<220>
<221> misc_feature
<223> popeye domain containing 2 (POPDC2)

<400> 9
cttctaggaa aggtggagtc ggtagtgaga agccggaggt gagaagaccc ctgagcggat 60

ggattcattc attttctgaa tttcctatgt gaggacagta ttagagccca gtgaggcttt 120

gagaggcccc aaagatgagc gccaacagca gcagagtggg ccagcttctc ttgcagggtt 180

cagcgtgcat taggtggaag caggatgtgg aaggggctgt ctaccaccta gccaactgcc 240

tcttactcct gggcttcatg gggggcagtg gggtgtatgg atgcttctat ctttttggct 300

tcctgagtgc aggttacctg tgctgcgtgc tgtggggctg gttcagtgcc tgtggcctgg 360

acattgttct ttggagcttc ctgctggctg tggtctgcct gctccagctg gcacacctgg 420

tataccgcct gcgtgaggac accctccctg aggagtttga cctcctctac aagacgctgt 480

gcctgccctt gcaggtgccc ctacagacat acaaggagat tgttcactgc tgcgaggagc 540

aggtcttaac tctggccact gaacagacct atgctgtgga gggtgagaca cccatcaacc 600

gcctgtccct gctgctctct ggccgggttc gtgtgagcca ggatgggcag tttctgcact 660

acatctttcc ataccagttc atggactctc ctgagtggga atcactacag ccttctgagg 720

agggggtgtt ccaggtcact ctgactgctg agacctcatg tagctacatt tcctggcccc 780

ggaaaagtct ccatcttctt ctgaccaaag agcgatacat ctcctgcctc ttctcggctc 840

tgctgggata tgacatctca gagaagctct acactctcaa tgacaagctc tttgctaagt 900

ttgggctgcg ctttgacatc cgccttccca gcctctacca tgtcctgggt cccactgctg 960

cagatgctgg accagagtcc gagaagggtg atgaggaagt ctgtgagcca gctgtgtccc 1020

ctcctcaggc cacacccacc tctctccagc aaacaccccc ttgttctacc cctccagcta 1080

ccaccaactt tcctgcacct cctacccggg ccaggttgtc caggccagac agtggcatac 1140

tggcttctag aattcctctc cagagctact ctcaagttat atccagggga caggcccctt 1200

tggctccaac ccacacgcct gaactttaag gatcattgga ctatcttctc tgtggccagc 1260

gcagctctct tctgtgttca cagaatggcc actgataggc atgcctcttt tcccacccac 1320

tggaaggctc acaggcaagg tgagagagga cacagaaggt gccaacactg tcgctacagt 1380

aaggacctga agtgactttg agaaattcac cctcacaaac cttccttcag gagcaggcat 1440

tggtagtgca gaggcacaga ttccgtcctt taccagctgc agaatcttgg gcaagttaca 1500

tagcctctgt gagcctcatc ggtaaacagt gggggttatg aaacccacct cacagggttg 1560

ttgtgaggat ccaatgagtt gatttaggta agcacctagc acatgccgtg gcaccaagta 1620

agcactcaat aaatcactca actcttaaaa aaaaaaaaaa aaaaaaa 1667

<210> 10
<211> 2032
<212> DNA
<213> Homo sapiens

<220>
<221> misc_feature
<223> tRNA selenocysteine 1 associated protein 1 (TRNAU1AP)

<400> 10
gtagcggtct ccgcagccag gattggcgcc cgcgcgggcc ttcgagcccc gcccgcagag 60

ccccgcccgc aaagccccac cccggtgcgc gggtatggcg gccagcctgt ggatgggcga 120

cctggaaccc tacatggatg agaacttcat ctccagagcc tttgccacca tgggggagac 180

cgtaatgagc gtcaaaatta tccgaaaccg cctcactggg atcccagctg gctactgctt 240

tgtagaattt gcagatttgg ccacagctga gaagtgtttg cataaaatta atgggaaacc 300

ccttccagga gccacacctt tacttagctt acagctgcac cagctggcac acctgggctc 360

atagaaccat ggagctggca gtgcccttag cggtcatccg tgcaaccccc tcattttata 420

caggagaaaa agctgaggct tagagagggg gagatgtttt ggccaaggcg aaacgtttta 480

aactgaacta tgccacttac gggaaacaac cagataacag ccctgagtat tccctctttg 540

tgggggacct gaccccggac gtggatgatg gcatgctgta tgaattcttc gtcaaagtct 600

acccctcctg tcggggaggc aaggtggttt tggaccagac aggcgtgtct aagggttatg 660

gttttgtgaa attcacagat gaactggaac agaagcgagc cctgacggag tgccagggag 720

cagtgggact ggggtctaag cctgtgcggc tgagcgtggc aatccctaaa gcgagccgtg 780

taaagccagt ggaatatagt cagatgtaca gttatagcta caaccagtat tatcagcagt 840

accagaacta ctatgctcag tggggctatg accagaacac aggcagctac agctacagtt 900

acccccagta tggctatacc cagagcacca tgcagacata tgaagaagtt ggagatgatg 960

cattggaaga ccccatgcca cagctggatg tgactgaggc caacaaggag ttcatggaac 1020

agagtgagga gctgtatgac gctctgatgg actgtcactg gcagcccctg gacacagtgt 1080

cttcagagat ccctgccatg atgtagccag gccaaaggac aagccaggtt gcatgatgtg 1140

agggagatga gagactcctt tttaaaaatt gtgaaacctt tttggaaata tgatttgtaa 1200

gattttaata atgactgttt ttggagatca tgaatgtttc tacaacactg ctgcattcat 1260

ttgaccattt gagtttgaag accaagggaa caacttttaa acaaggttca aattggtttc 1320

cttcacagga atcctttgtc caggtagtta tccacatagg acagtttggg attatccaaa 1380

actgggatga gcagcttggg ataattcaca cactaaagcc tgagttcaag gtttctcagt 1440

tgacaggtag gtatgtaaat cagggttctc tggagtattt gtaggacctg ccatatttga 1500

gtcctaactt ggtgcaagtc tggcagctag gtagaaaagg cactctagca ggtgtggtag 1560

gagcacgccc cagaaggtag aatgtggtgg ggtgtgatgg aacaggccag ttggcctaag 1620

aattagtact tgctctaata atgggtttca tctattttca tgagttgcag catctgcata 1680

tacaaggcct ggatcacttt aggatcaata ttttgacacg ggagaaggca gctcagaaag 1740

gattaaggaa gatagctcaa agccaaagca gaccctccgt gtgaacttta tactgctcac 1800

ttccgtgttt cccaagtaaa tggggaaact gtccaacctt gtcatgtgtt gggacttact 1860

gcttgaggca aagcattcat cctacatcat aaaaaatctc caaagatctc tttaagggct 1920

gggcatggtg gctcactttg ggagatggat tgcttgagct cgggaagaga ccagttcggt 1980

ctctacaaaa aaatacaaac acaaatatac ttttaaaaaa aaaaaaaaaa aa 2032

<210> 11
<211> 1375
<212> DNA
<213> Homo sapiens

<220>
<221> misc_feature
<223> regulator of G-protein signaling 2, 24kDa (RGS2)

<400> 11
gcaaacagcc ggggctccag cgggagaacg ataatgcaaa gtgctatgtt cttggctgtt 60

caacacgact gcagacccat ggacaagagc gcaggcagtg gccacaagag cgaggagaag 120

cgagaaaaga tgaaacggac cctttttaaa gattggaaga cccgtttgag ctacttctta 180

caaaattcct ctactcctgg gaagcccaaa accggcaaaa aaagcaaaca gcaagctttc 240

atcaagcctt ctcctgagga agcacagctg tggtcagaag catttgacga gctgctagcc 300

agcaaatatg gtcttgctgc attcagggct tttttaaagt cggaattctg tgaagaaaat 360

attgaattct ggctggcctg tgaagacttc aaaaaaacca aatcacccca aaagctgtcc 420

tcaaaagcaa ggaaaatata tactgacttc atagaaaagg aagctccaaa agagataaac 480

atagattttc aaaccaaaac tctgattgcc cagaatatac aagaagctac aagtggctgc 540

tttacaactg cccagaaaag ggtatacagc ttgatggaga acaactctta tcctcgtttc 600

ttggagtcag aattctacca ggacttgtgt aaaaagccac aaatcaccac agagcctcat 660

gctacatgaa atgtaaaagg gagcccagaa atggaggaca tttcattctt tttcctgagg 720

ggaaggactg tgacctgcca taaagactga ccttgaattc agcctgggtg ttcaggaaac 780

atcactcaga actattgatt caaagttggg tagtgaatca ggaagccagt aactgactag 840

gagaagctgg tatcagaaca gcttccctca ctgtgtacag aacgcaagaa gggaataggt 900

ggtctgaacg tggtgtctca ctctgaaaag caggaatgta agatgatgaa agagacaatg 960

taatactgtt ggtccaaaag catttaaaat caatagatct gggattatgt ggccttaggt 1020

agctggttgt acatctttcc ctaaatcgat ccatgttacc acatagtagt tttagtttag 1080

gattcagtaa cagtgaagtg tttactatgt gcaacggtat tgaagttctt atgaccacag 1140

atcatcagta ctgttgtctc atgtaatgct aaaactgaaa tggtccgtgt ttgcattgtt 1200

aaaaatgatg tgtgaaatag aatgagtgct atggtgttga aaactgcagt gtccgttatg 1260

agtgccaaaa atctgtcttg aaggcagcta cactttgaag tggtctttga atacttttaa 1320

taaatttatt ttgataaata atattgaaca aaaaaaaaaa aaaaaaaaaa aaaaa 1375

<210> 12
<211> 211
<212> PRT
<213> Homo sapiens

<220>
<221> misc_feature
<223> RGS2

<400> 12

Met Gln Ser Ala Met Phe Leu Ala Val Gln His Asp Cys Arg Pro Met
1               5                   10                  15

Asp Lys Ser Ala Gly Ser Gly His Lys Ser Glu Glu Lys Arg Glu Lys
            20                  25                  30

Met Lys Arg Thr Leu Leu Lys Asp Trp Lys Thr Arg Leu Ser Tyr Phe
        35                  40                  45

Leu Gln Asn Ser Ser Thr Pro Gly Lys Pro Lys Thr Gly Lys Lys Ser
    50                  55                  60

Lys Gln Gln Ala Phe Ile Lys Pro Ser Pro Glu Glu Ala Gln Leu Trp
65                  70                  75                  80

Ser Glu Ala Phe Asp Glu Leu Leu Ala Ser Lys Tyr Gly Leu Ala Ala
                85                  90                  95

Phe Arg Ala Phe Leu Lys Ser Glu Phe Cys Glu Glu Asn Ile Glu Phe
            100                 105                 110

Trp Leu Ala Cys Glu Asp Phe Lys Lys Thr Lys Ser Pro Gln Lys Leu
        115                 120                 125

Ser Ser Lys Ala Arg Lys Ile Tyr Thr Asp Phe Ile Glu Lys Glu Ala
    130                 135                 140

Pro Lys Glu Ile Asn Ile Asp Phe Gln Thr Lys Thr Leu Ile Ala Gln
145                 150                 155                 160

Asn Ile Gln Glu Ala Thr Ser Gly Cys Phe Thr Thr Ala Gln Lys Arg
                165                 170                 175

Val Tyr Ser Leu Met Glu Asn Asn Ser Tyr Pro Arg Phe Leu Glu Ser
            180                 185                 190

Glu Phe Tyr Gln Asp Leu Cys Lys Lys Pro Gln Ile Thr Thr Glu Pro
        195                 200                 205

His Ala Thr
    210

<210>  13
<211>  108
<212>  PRT
<213>  Homo sapiens

<220>
<221>  misc_feature
<223>  POLR3K

<400>  13

Met Leu Leu Phe Cys Pro Gly Cys Gly Asn Gly Leu Ile Val Glu Glu
1               5                   10                  15

Gly Gln Arg Cys His Arg Phe Ser Cys Asn Thr Cys Pro Tyr Val His
            20                  25                  30

Asn Ile Thr Arg Lys Val Thr Asn Arg Lys Tyr Pro Lys Leu Lys Glu
        35                  40                  45

Val Asp Asp Val Leu Gly Gly Ala Ala Ala Trp Glu Asn Val Asp Ser
    50                  55                  60

Thr Ala Glu Ser Cys Pro Lys Cys Glu His Pro Arg Ala Tyr Phe Met
65                  70                  75                  80

Gln Leu Gln Thr Arg Ser Ala Asp Glu Pro Met Thr Thr Phe Tyr Lys
                85                  90                  95

Cys Cys Asn Ala Gln Cys Gly His Arg Trp Arg Asp
            100                 105

<210>  14
<211>  823
<212>  PRT
<213>  Homo sapiens

<220>
<221>  misc_feature
<223>  MERTK

<400>  14


<210>  15
<211>  999
<212>  PRT
<213>  Homo sapiens


<220>
<221>  misc_feature
<223>  MERTK precursor

<400>  15

<210>  16
<211>  21

<212>  DNA
<213>  artificial

<220>
<223>  MERTK forward primer

<400>  16
attcccgatt ggagacagga c                                                21


<210>  17
<211>  21
<212>  DNA
<213>  artificial

<220>
<223>  MERTK reverse primer

<400>  17
tccattccca gggcaatatc c                                                21


<210>  18
<211>  21
<212>  DNA
<213>  artificial

<220>
<223>  CUL4B forward primer

<400>  18
gtcggaaaga gtgcatctgt a                                                21


<210>  19
<211>  20
<212>  DNA
<213>  artificial

<220>
<223>  CUL4B reverse primer

<400>  19
caagtttgct ggtgaaagca                                                  20


<210>  20
<211>  20
<212>  DNA
<213>  artificial

<220>
<223>  LGALS9 forward primer

<400>  20
ccaggacgga cttcagatca                                                  20


<210>  21
<211>  20
<212>  DNA
<213>  artificial

<220>
<223>  LGALS9 reverse primer

<400>  21
ccactgaagc cagtctgaaa                                                    20


<210>  22
<211>  22
<212>  DNA
<213>  artificial

<220>
<223>  NFYB forward primer

<400>  22
gctttgacag ttatgtggaa cc                                                 22


<210>  23
<211>  19
<212>  DNA
<213>  artificial

<220>
<223>  NFYB reverse primer

<400>  23
tgactgctcc accaattcc                                                     19


<210>  24
<211>  22
<212>  DNA
<213>  artificial

<220>
<223>  PLCB1 forward primer

<400>  24
acttaagctg caagtcactc ca                                                 22


<210>  25
<211>  20
<212>  DNA
<213>  artificial

<220>
<223>  PLCB1 reverse primer

<400>  25
tcgcttccga tctgctgaaa                                                    20


<210>  26
<211>  20
<212>  DNA
<213>  artificial

<220>
<223>  SLC2A1 forward primer

<400>  26
tccctgcagt ttggctacaa                                                   20


<210>  27
<211>  22
<212>  DNA
<213>  artificial

<220>
<223>  SLC2A1 reverse primer

<400>  27
ggacccatgt ctggttgtag aa                                                22


<210>  28
<211>  20
<212>  DNA
<213>  artificial

<220>
<223>  AAGAB forward primer

<400>  28
agatgcccag gttgatagca                                                   20


<210>  29
<211>  21
<212>  DNA
<213>  artificial

<220>
<223>  AAGAB reverse primer

<400>  29
ctccagtggt aagactggct a                                                 21


<210>  30
<211>  20
<212>  DNA
<213>  artificial

<220>
<223>  POLR3K forward primer

<400>  30
ccctacgtgc acaacatcac                                                   20


<210>  31
<211>  20
<212>  DNA
<213>  artificial

<220>
<223>  POLR3K reverse primer

<400>  31
tccaccaagc acatcatcca                                                   20

<210>  32
<211>  21
<212>  DNA
<213>  artificial

<220>
<223>  POPDC2 forward primer

<400>  32
gagggtgaga cacccatcaa c                                                21


<210>  33
<211>  21
<212>  DNA
<213>  artificial

<220>
<223>  POPDC2 reverse primer

<400>  33
tagtgcagaa actgcccatc c                                                21


<210>  34
<211>  20
<212>  DNA
<213>  artificial

<220>
<223>  TRNAU1AP forward primer

<400>  34
aaggtggttt tggaccagac                                                  20


<210>  35
<211>  20
<212>  DNA
<213>  artificial

<220>
<223>  TRNAU1AP reverse primer

<400>  35
ctcgcttctg ttccagttca                                                  20


<210>  36
<211>  24
<212>  DNA
<213>  artificial

<220>
<223>  RGS2 forward primer

<400>  36
cagaacgcaa gaagggaata ggtg                                             24


<210>  37
<211>  23

<212>  DNA
<213>  artificial

<220>
<223>  RGS2 reverse primer

<400>  37
tttggcactc ataacggaca ctg                                               23